# EUROPEAN PATENT SPECIFICATION

(11) **EP 2 215 247 B1**
(45) Date of publication and mention of the grant of the patent: **24.09.2014**
(21) Application number: 08849056.0
(22) Date of filing: 12.11.2008
(51) Int. Cl.: C07K 16/28, C12N 15/82

(54) **PRODUCTION OF CYTOTOXIC ANTIBODY-TOXIN FUSION IN EUKARYOTIC ALGAE**
HERSTELLUNG EINER FUSION AUS ZYTOTOXISCHEM ANTIKÖRPER UND TOXIN BEI EUKARYOTISCHEN ALGEN
PRODUCTION DE FUSION ANTICORPS-TOXINE CYTOTOXIQUE DANS UNE ALGUE EUCARYOTIQUE

(30) Priority: 13.11.2007 US 987726 P
(43) Date of publication of application: 11.08.2010
(73) Proprietor: The Scripps Research Institute, La Jolla, CA 92037 (US)
(72) Inventor: MAYFIELD, Stephen, P., Cardiff-by-the-Sea, CA 92007 (US); TRAN, Miller, La Jolla, 92037 CA (US)
(74) Representative: Care, Alison
(86) International application number: PCT/US2008/083282
(87) International publication number: WO 2009/064815

(56) References cited:
- WO-A1-2005/052007
- WO-A2-01/31006
- WO-A2-01/87982
- WO-A2-02/06497
- WO-A2-03/051926
- WO-A2-03/064992
- WO-A2-03/091413
- WO-A2-2006/061723
- WO-A2-2007/085470
- US-A- 6 004 552
- US-A- 6 046 310
- US-A1- 2004 014 174
- US-A1- 2004 142 415
- US-A1- 2006 030 000
- US-A1- 2006 153 860
- STAUB J M ET AL: "HIGH-YIELD PRODUCTION OF A HUMAN THERAPEUTIC PROTEIN IN TOBACCO CHLOROPLASTS" NATURE BIOTECHNOLOGY, NATURE PUBLISHING GROUP, NEW YORK, NY, US LNKD- DOI:10.1038/73796, vol. 18, no. 3, 1 January 2000 (2000-01-01), pages 333-338, XP000887246 ISSN: 1087-0156
- MAYFIELD STEPHEN P ET AL: "Expression and assembly of a fully active antibody in algae" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0237108100, vol. 100, no. 2, 21 January 2003 (2003-01-21), pages 438-442, XP002440629 ISSN: 0027-8424
- BOEHM ROBERT: "Bioproduction of therapeutic proteins in the 21st century and the role of plants and plant cells as production platforms." ANNALS OF THE NEW YORK ACADEMY OF SCIENCES APR 2007 LNKD- PUBMED:17470916, vol. 1102, April 2007 (2007-04), pages 121-134, XP002601710 ISSN: 0077-8923
- KIPRIYANOV S M ET AL: "Generation and production of engineered antibodies" MOLECULAR BIOTECHNOLOGY, HUMANA PRESS, INC, US LNKD- DOI:10.1385/MB:26:1:39, vol. 26, no. 1, 1 January 2004 (2004-01-01), pages 39-60, XP009044299 ISSN: 1073-6085
- WANG D ET AL: "Generation and characterization of an anti-CD19 single-chain Fv immunotoxin composed of C-terminal disulfide-linked dgRTA" BIOCONJUGATE CHEMISTRY, ACS, WASHINGTON, DC, US LNKD- DOI:10.1021/BC970071W, vol. 8, no. 6, 1 November 1997 (1997-11-01), pages 878-884, XP002445345 ISSN: 1043-1802

## Description

### BACKGROUND OF THE INVENTION

### FIELD OF THE INVENTION

The present invention relates generally to methods and compositions for expressing polypeptides in chloroplasts, and more specifically to antibody-toxin fusion constructs that encode therapeutic products that are expressed in chloroplasts.

### BACKGROUND INFORMATION

Protein based therapeutics, or biologics, are the fastest growing sector of drug development, mainly due to the efficacy and specificity of these molecules. The specificity of biologies comes from their complexity, and because biologics are only produced in living cells, the production of these molecules can be time consuming and expensive.

Previous monoclonal antibody-based therapies have been developed in which antibody binding to cell surface proteins results in activation of antibody-dependent cell-mediated cytotoxicity (ADCC) or antibody-toxin conjugates are used that are capable of directly killing targeted cells. In ADCC, for example, an immune response is activated where antibodies coat a target cell thereby marking them for attack by natural killer (NK) cells. Therapeutics based on ADCC have been shown to be effective for the treatment of several types of cancers. For fusions of antibodies and antibody fragments to chemical or protein toxins that involve direct killing targeted cells, such immunotoxins are usually constructed by chemically conjugating a cell toxic agent to an antibody directed against a cell surface protein known to internalize after antibody binding. Once internalized within the cell, the toxin is able to disrupt a vital cellular function such protein synthesis, leading to death of the target cell.

Although the utility of these types of molecules seems to have many applications, only one cancer drug using this strategy is presently on the market (Mylotarg®, Wyeth-Ayerst Laboratories). One reason for the failure of these hybrid molecules to be utilized more often may lie in the complex nature of their construction, with the antibody half of the molecule typically produced in mammalian cells and the toxin half of the molecule produced in bacteria or by chemical synthesis, followed by chemically linking of the two parts to one another in ratios of one or more toxin moieties per antibody. Chemically coupling a small molecule or protein toxin to an antibody suffers from several limitations. First, there are limited chemistries available for coupling of a small molecule or protein toxin to an antibody, and by the efficiencies of these chemical reactions. Second, the chemical coupling can be limited by the availability of suitable sites for attachment to the antibody, potentially resulting in the production of antibody-toxin fusions where the antibody portion of the molecule is rendered inactive. Once administered, the coupled toxin could dissociate prematurely from the antibody prior to internalization resulting in off-target cytotoxicity and reduced cell-killing at the target site by competition of the uncoupled antibody for cell surface binding sites with intact antibody-toxin conjugates.

An alternative approach for the production of antibody-toxin conjugates is the construction of genetic fusions, where an antibody coding region is genetically linked to the coding region of a protein toxin. Production of these types of fusion proteins is strictly limited to prokaryotic expression systems, as an active immunotoxin would kill any susceptible eukaryotic host. There are other limitations to this type of approach as well, because prokaryotic systems are typically unable to express full-length antibodies, and even the production of antibody fragments, such as scFvs and Fabs as fusions, fused to protein toxin domains is problematic as these domains are often insoluble in *E. coli* expression systems. This insolubility results in poor yields of active molecules and in time consuming and expensive protocols for solubilizing and re-folding of aggregated proteins from bacterial inclusion bodies.

Many protein-based eukaryotic toxins target the translational machinery of eukaryotic cells, specifically the 80S ribosome and cytoplasmic translational initiation and elongation factors. Protein toxins can be produced in prokaryotes because the translation machinery of bacteria is substantially different than that of eukaryotic cells in general. In a similar fashion, the translational apparatus of plant and algal plastids is fundamentally different from the translation machinery in other eukaryotic cytoplasm. Plastids contain prokaryotic-like 70S ribosome and associated translational factors that are very different from those present in the typical eukaryotic cytosol. Consequently, the chloroplast presents a unique environment for the production of eukaryotic toxins and for the production of antibody-toxin fusions, as plastids have evolved to contain a suite of molecular chaperones and redox factors capable of modulating complex protein folding and assembly, including formation of disulfide bonds.

By generating antibody-toxin fusion proteins as genetic fusions, instead of as chemical fusions, the production of these complex molecules can be greatly facilitated, making it possible to produce immunotoxin molecules with superior properties.

The expression of biologics in algae offers an attractive alternative to traditional mammalian-based expression systems, as the production of proteins in algae has inherently low costs of capitalization and production, and stable transgenic lines can be generated in a short period of time.

### SUMMARY OF THE INVENTION

The present invention provides as a first aspect a nucleic acid construct comprising in operable linkage:
a) nucleic acid signaling elements for homologous recombination and expression of a fusion protein in a chloroplast of a plant or eukaryotic alga; and
b) a first polynucleotide sequence encoding a first polypeptide and a second polynucleotide sequence encoding a toxin functional in a eukaryotic cell, wherein the first polypeptide encodes a binding domain specific for a cluster of differentiation "CD" cell surface marker, and wherein the first and second polynucleotide sequences are expressed as a fusion protein in the chloroplast of the plant or eukaryotic alga, wherein the toxin is a bacterial or plant derived toxin.

The binding domain may comprise an antibody or an antigen binding fragment thereof, and, the antibody or the antigen binding fragment thereof may be selected from a complete antibody, and an Fc region.

The toxin may be an endotoxin or an exotoxin. The toxin may be exotoxin A or gelonin.

The cell surface marker may be selected from the group consisting of, CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d, and CD66e.

The present invention also provides as a second aspect a plant or algal chloroplast comprising the construct according to the first aspect.

Also provided, as a third aspect is a plant or algal cell comprising the chloroplast of the second aspect.

As a fourth aspect, the invention provides a method for producing a functional fusion protein comprising,
a) contacting a chloroplast of a plant or eukaryotic alga with at least one expression construct, the expression construct comprising in operable linkage, (i) nucleic acid signal elements for homologous recombination and expression of the fusion protein in the chloroplast of the plant or eukaryotic alga, and (ii) a first polynucleotide sequence encoding a first polypeptide comprising a binding domain specific for a cluster of differentiation "CD" cell surface marker, and a second polynucleotide sequence encoding a toxin functional in a eukaryotic cell, the toxin being a bacterial or plant derived toxin;
b) allowing the construct to integrate into the chloroplast genome; and
c) expressing the first and second polynucleotide sequences as a fusion protein in the chloroplast of the plant or eukaryotic alga.

The binding domain may comprise an antibody or an antigen binding fragment thereof, wherein the antibody or the antigen binding fragment thereof may be selected from a complete antibody, and an Fc region.

The toxin may be an endotoxin or an exotoxin. The toxin may be exotoxin A or gelonin.

The cell surface marker of the fourth aspect may be selected from the group consisting of CD1, CD2, CD3, CD4, CD5, CD6, CD7, CD8, CD9, CD10, CD11a, CD11b, CD11c, CD11d, CDw12, CD13, CD14, CD 15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d, and CD66e.

The method of the fourth aspect may further comprise:
d) isolating the fusion protein from the plant or eukaryotic alga.

### BRIEF DESCRIPTION OF THE DRAWINGS

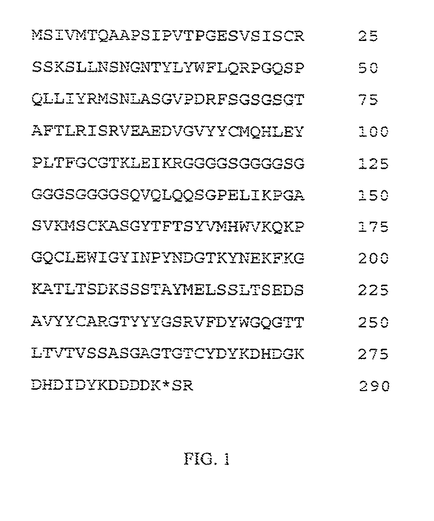

Figure 1 shows the amino acid sequence of the anti-CD 19 single chain antibody (SEQ ID NO:1). The sequence was derived from a mouse anti-human CD 19 antibody. Amino acid residues 1 to 114 define the variable regions of the light chain, amino acid residues 115 to 134 define a flexible peptide linker, amino acid residues 135 to 263 define the variable region of the heavy chain, and amino acid residues 264 to 290 define the FLAG epitope tag.

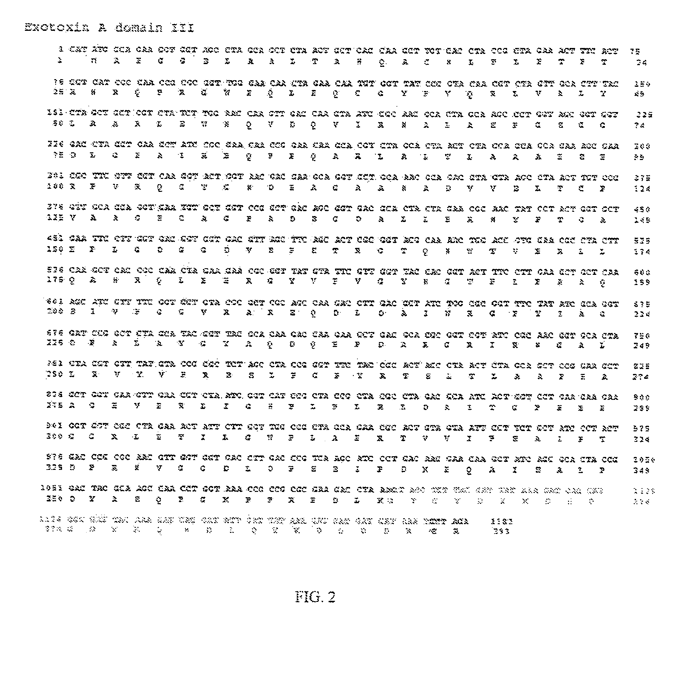

Figure 2 shows the nucleotide and amino acid sequences of domains II and III from exotoxin A of *Pseudomonas* (SEQ ID NOS:2 and 3, respectively). Amino acid residues 1 to 364 define the catalytic and translocation domain II and III, while amino acid residues 365 to 391 indicate the FLAG epitope tag.

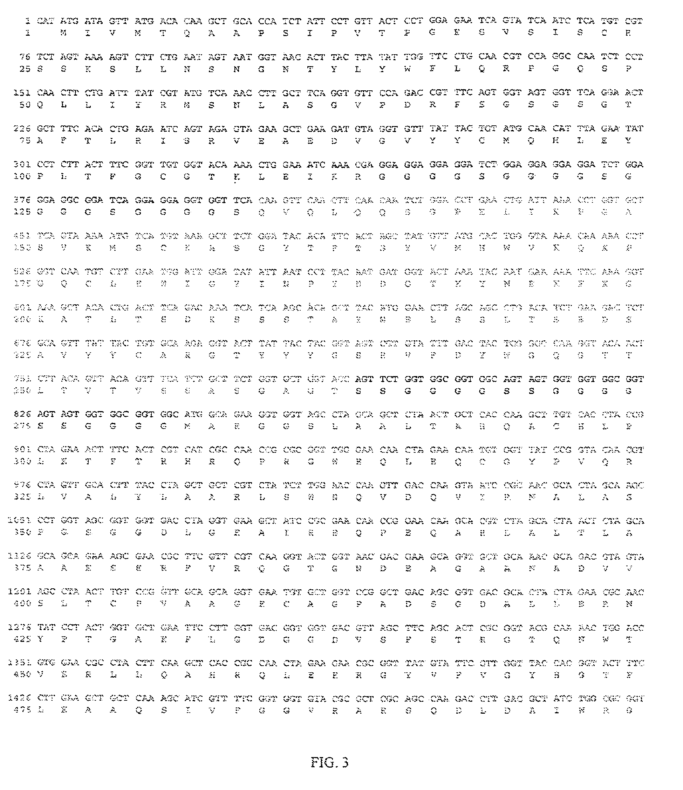

Figure 3 shows the nucleotide and amino acid sequences of CD19 scFv-exotoxin A fusion protein (SEQ ID NOS:4 and 5, respectively). Amino acid residues 1 to 113 define the variable regions of the light chain, amino acid residues 114 to 133 and 263 to 280 define flexible peptide linkers and amino acid residues 134 to 260 define the variable region of the heavy chain. Exotoxin A domains II and III are defined by amino acid residues 281 to 644 and amino acid residues 645 to 671 define the FLAG epitope tag.

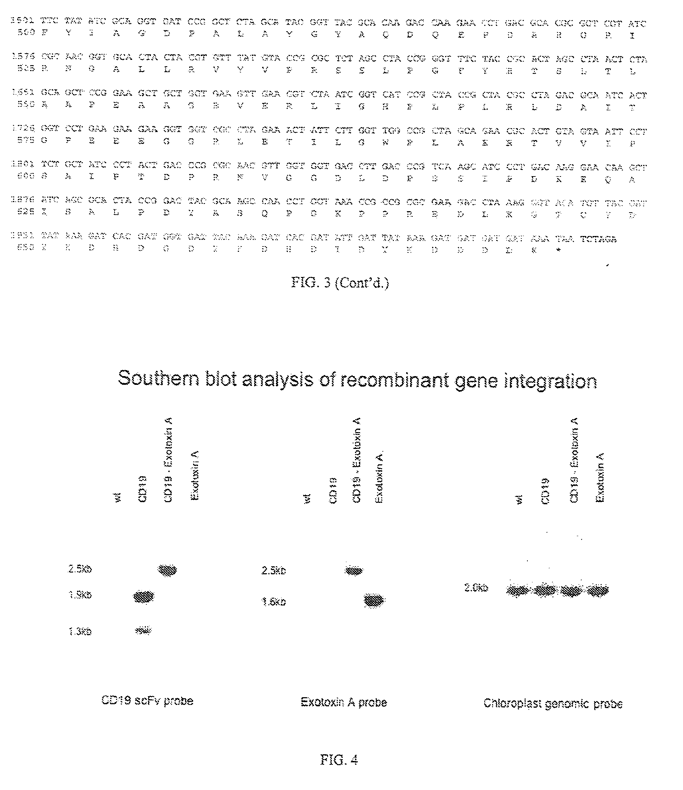

Figure 4 shows the Southern blot analysis of *C*. *reinhardtii* transgenic lines containing, CD 19 scFv, CD19-exotoxin A, and Exotoxin A. Blots were probed with a CD19 scFv cDNA (left panel), an ETA domains II and III probe (central panel), or a chloroplast genomic fragment (right panel).

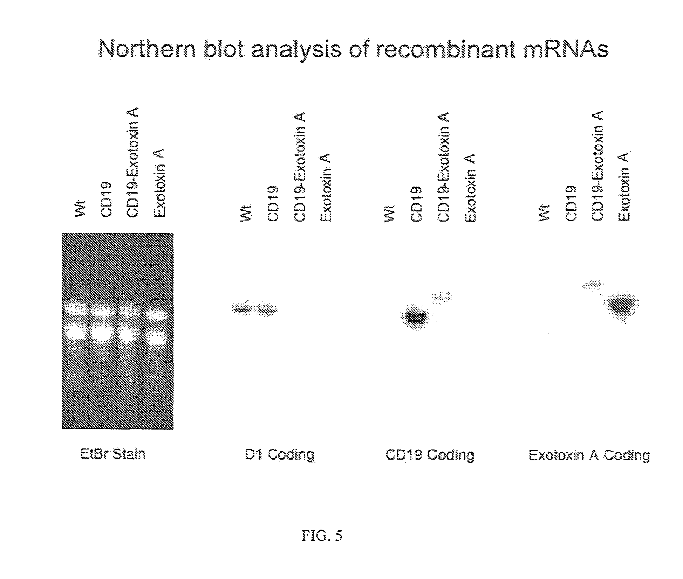

Figure 5 shows a Northern blot analysis of recombinant mRNA accumulation in three transgenic lines. Total RNA was separated on denaturing agarose gels and stained with ethidium bromide (left panel, or blotted to membranes and hybridized with D1, exotoxin A, or CD 19 scFv coding region.

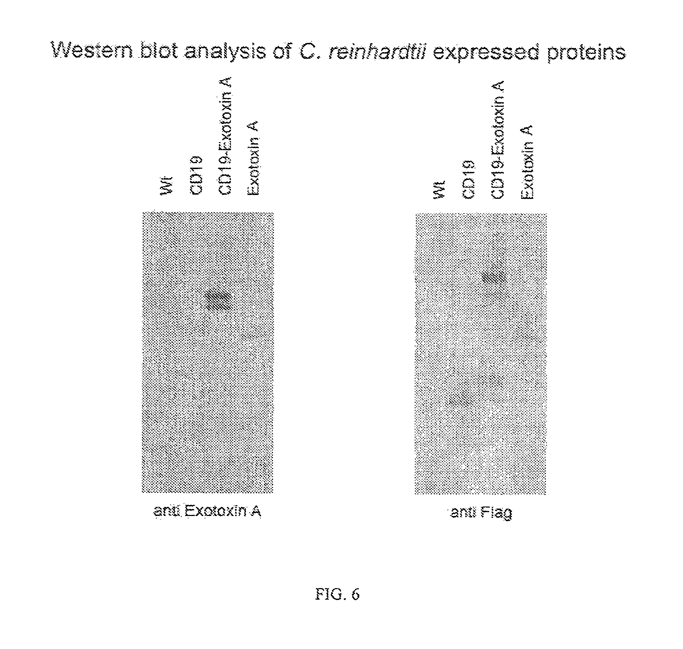

Figure 6 shows a Western blot analysis of recombinant protein accumulation in *C*. *reinhardtii* transgenic lines. Total proteins from wt and transgenic lines were blotted to membranes and decorated with anti-exotoxin A (left panel) or anti-FLAG (right panel) antisera.

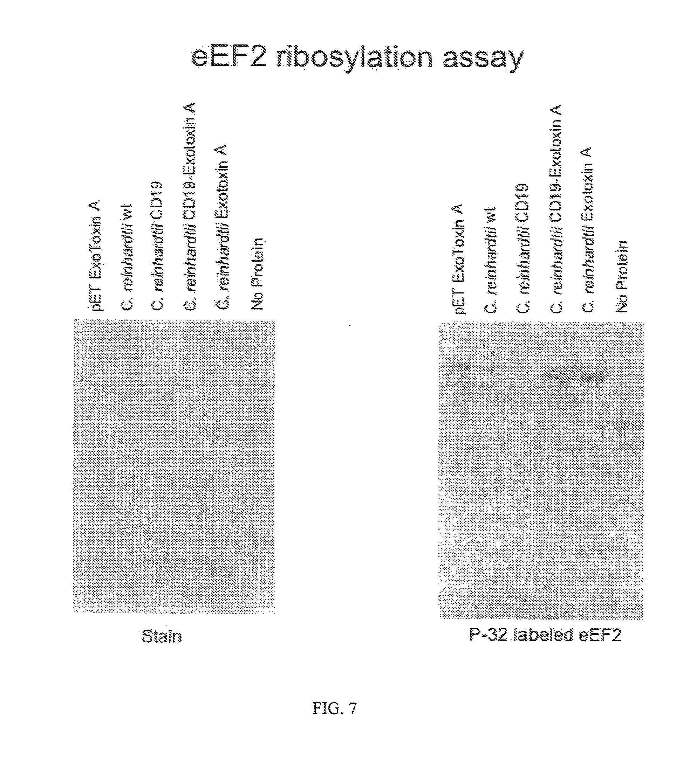

Figure 7 shows an exotoxin A domain III ribosylation activity assay. Exotoxin A specifically ribosylates eukaryotic elongation factor 2 (eEF2). Equal amounts of eEF2 were incubated with bacterial expressed exotoxin A domains II and III (pET exotoxin A), or with *C*. *reinhardtii* protein extracts from wt, a transgenic line expressing CD 19 scFv alone, CD19-exotoxin A fusion protein, exotoxin A domain III, or no protein. The left panel shows a stain gel of the proteins after separation by SDS-PAGE.

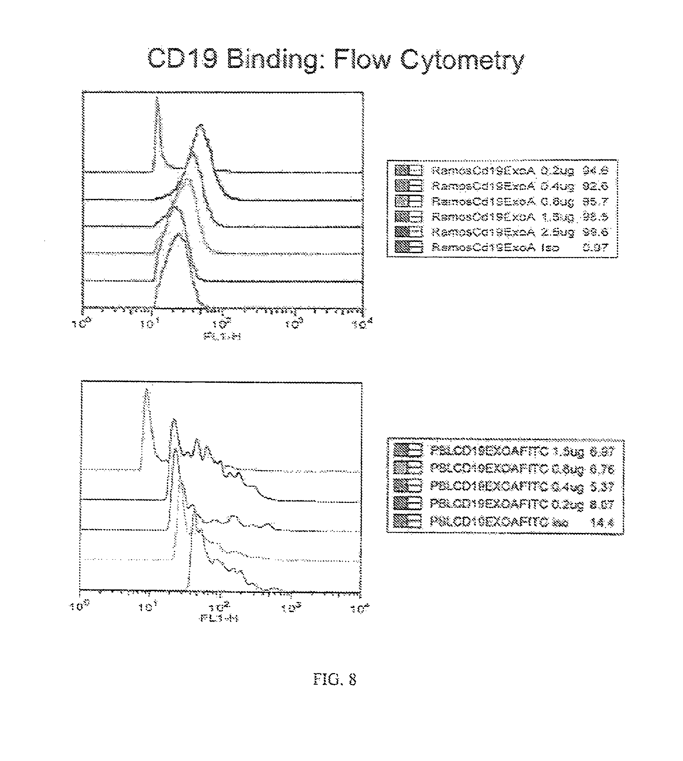

Figure 8 illustrates the binding of CD19-ETA to CD 19 positive B-cells. Top panel shows fluorescence of Ramos B-cells incubated with increasing concentrations of CD 19-ETA-flag and a FITC labeled anti-flag antibody. The highest concentration being represented by the second line from the top of the graph with the control represented by the top most line. The lower panel shows human peripheral blood lymphocytes (PBL) labeled with the same CD19-ETA-Flag and FITC labeled anti-Flag as in the top panel. The highest concentration being shown by the bottom most line of the graph with the control represented by the top most line.

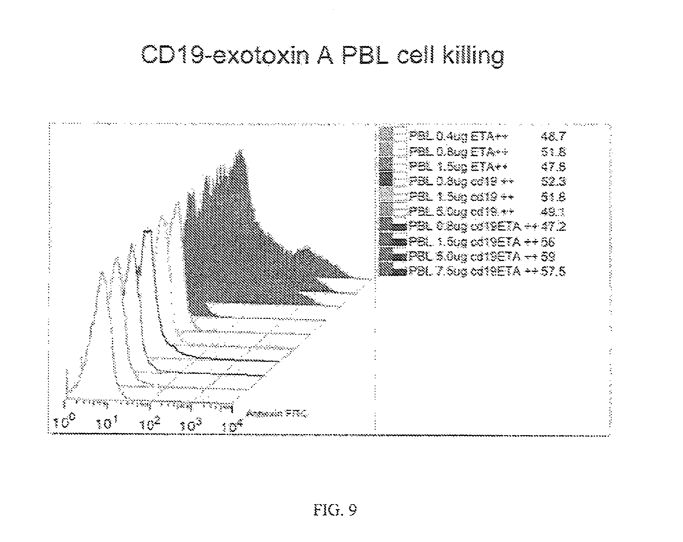

Figure 9 shows PBL cell viability after treatment with exotoxin A alone (lines 1-3 from the bottom of the graph), CD 19 antibody alone (lines 4-6 from the bottom of the graph), or CD 19-ETA antibody toxin fusion (lines 7-10 from the bottom of the graph). Cells were stained with anti-annexin PE.

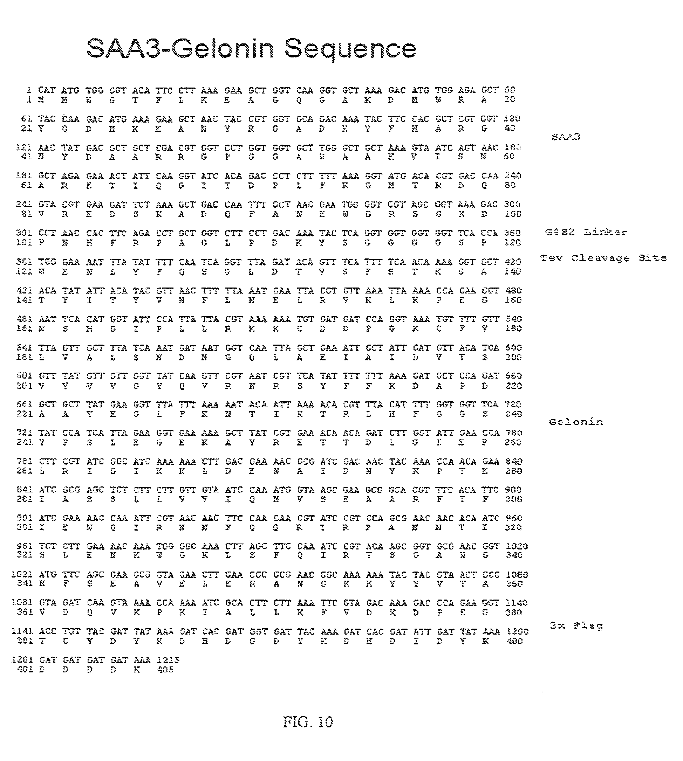

Figure 10 shows the nucleotide and amino acid sequences of the SAA-nGelonin fusion protein (SEQ ID NOS:6 and 7, respectively). Amino acid residues 1 to 113 define the codon optimized bovine serum amyloid A 3 protein, amino acid residues 114 to 119 define the flexible peptide linker, amino acid residues 120 to 128 define a TEV protease site, amino acid residues 129 to 379 define native Gelonin, and amino acid residues 380 to 405 at the carboxy terminus define the FLAG epitope tag.

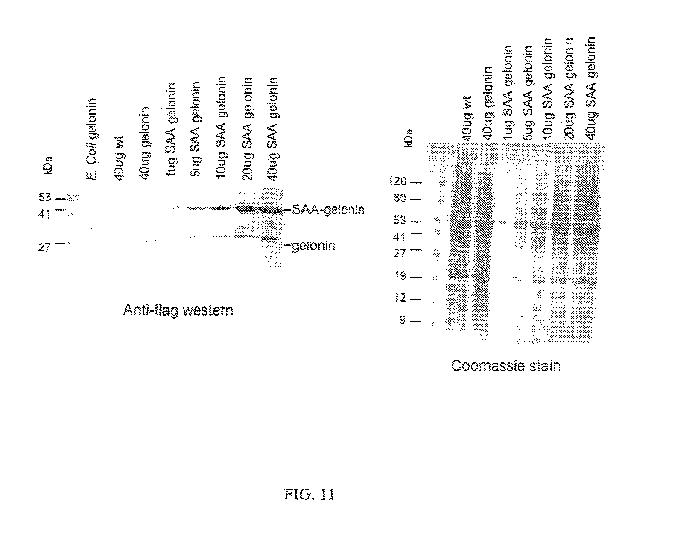

Figure 11 shows a Western blot analysis of recombinant rGelonin and SAA-nGelonin protein accumulation in *C*. *reinhardtii* transgenic chloroplasts. Total proteins from wt, a transgenic line expressing rGel and a dilution series of proteins from a transgenic line expressing SAA-nGelonin are shown. The proteins were blotted to membranes and decorated with anti-FLAG (right panel) antisera.

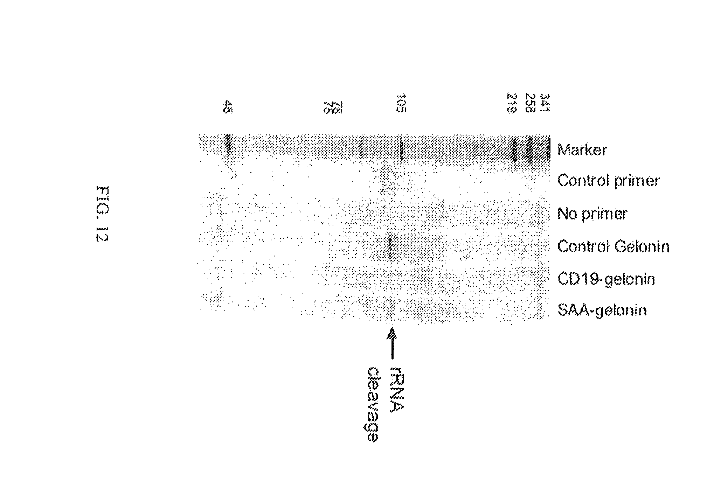

Figure 12 shows an *in vitro* activity assay of isolated chloroplast expressed SAA-nGelonin. Lane 2 shows a control primer extension product. Lane 3 shows primer extension with no added protein, lane 4 shows primer extension with bacterially expressed rGelonin added, lane 6 shows primer extension with purified SAA-nGelonin added.

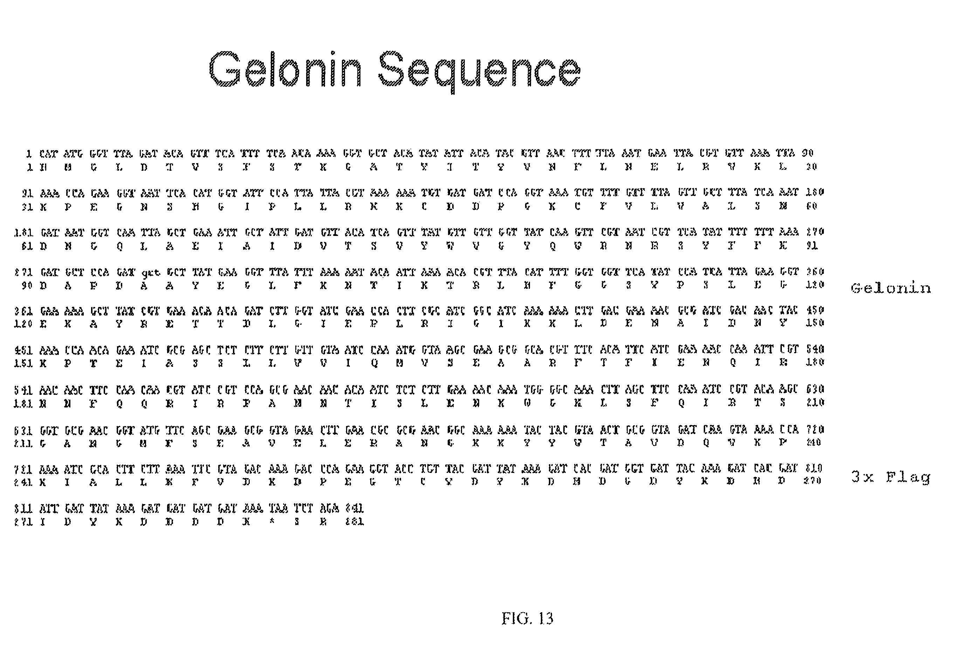

Figure 13 shows nucleotide and amino acid sequences of the native gelonin sequence linked to FLAG epitope tag (SEQ ID NOS:8 and 9, respectively). Amino acid residues 1 to 253 define native Gelonin, and amino acid residues 254 to 281 at the carboxy terminus define the FLAG epitope tag.

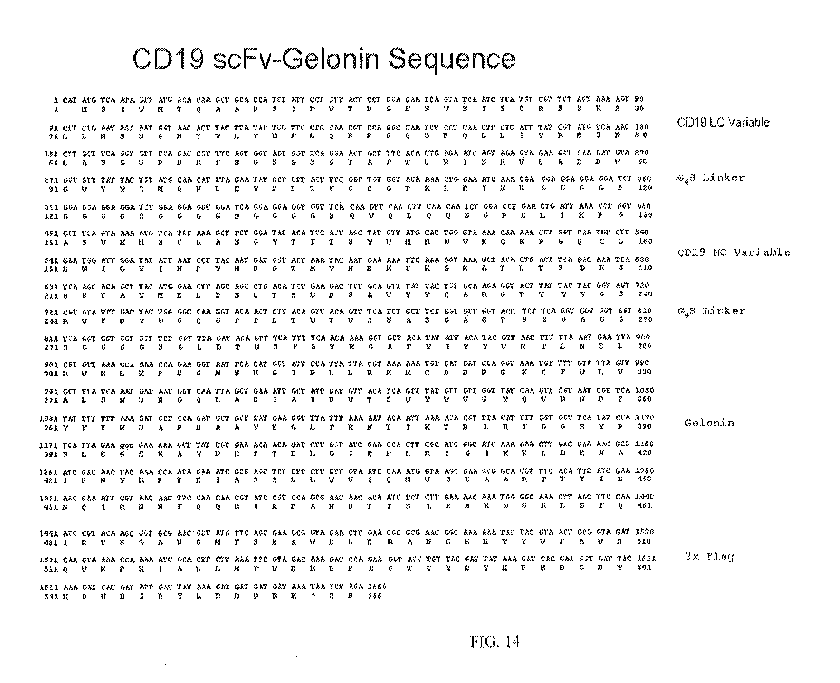

Figure 14 shows the nucleotide and amino acid sequences of the CD 19 scFv-Gelonin fusion protein (SEQ ID NOS:10 and 11, respectively). Amino acid residues 1 to 115 define the variable regions of the light chain, amino acid residues 116 to 135 define the flexible peptide linker, amino acid residues 136 to 264 define the variable region of the heavy chain, amino acid residues 265 to 276 define the flexible peptide linker, amino acid residues 277 to 527 define native Gelonin, and amino acid residues 528 to 556 at the carboxy terminus define the FLAG epitope tag.

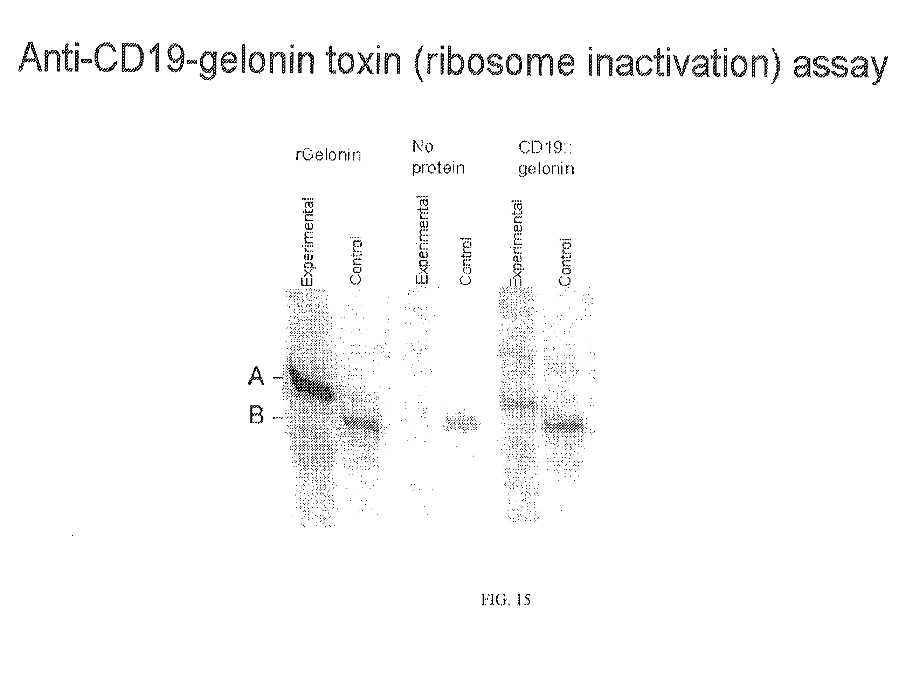

Figure 15 shows an *in vitro* gelonin assay using the CD 19 scFv-Gelonin fusion protein. Gelonin activity is assayed by primer extension with radio-labeled primer. Yeast ribosomes were treated with purified recombinant gelonin, CD19:Gelonin, or untreated (no protein). Active gelonin will cleave the rRNA within the ricin loop. After treatment rRNA is isolated and used as a template for primer extension. 'Experimental' primers will give a product if gelonin activity is present (Figure 15A). 'Control' primers will give a product (Figure 15B) if rRNA is present.

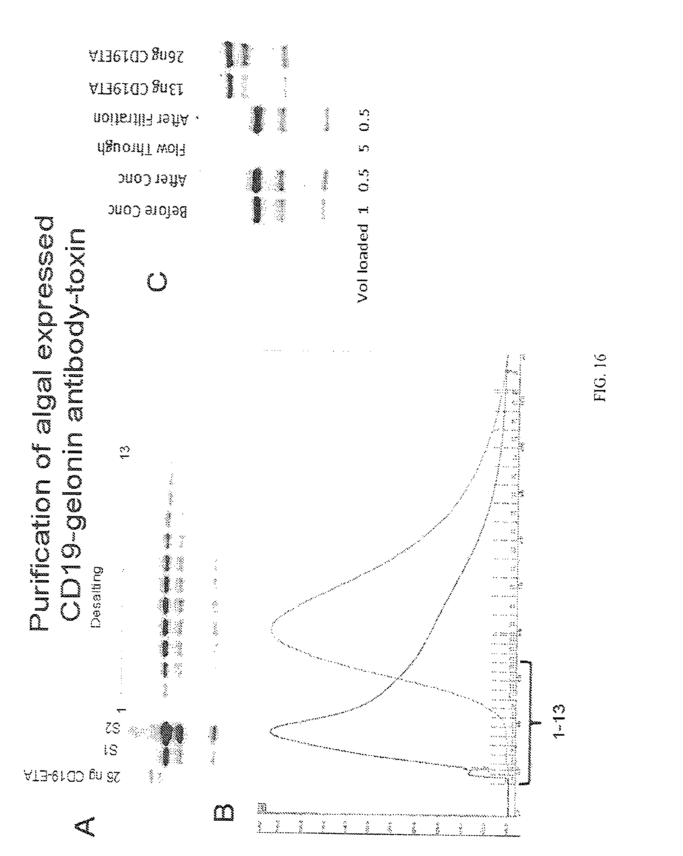

Figure 16 shows various experiments using the CD19 scFv-Gelonin fusion protein. Figure 16A shows a Western blot of starting material, purified by FLAG affinity from crude algae lysate, before and after concentration (S1 and S2 respectively), then elutions from desalting column. Figure 16B shows the elution profile from desalting column. Darker line shows UV absorbance, lighter line shows conductivity (salt). Figure 16C shows a Western blot of purified desalted samples. Elutions 2-10 from desalting column were pooled (lane 1) and concentrated (lane 2), and filtered (lane 4).

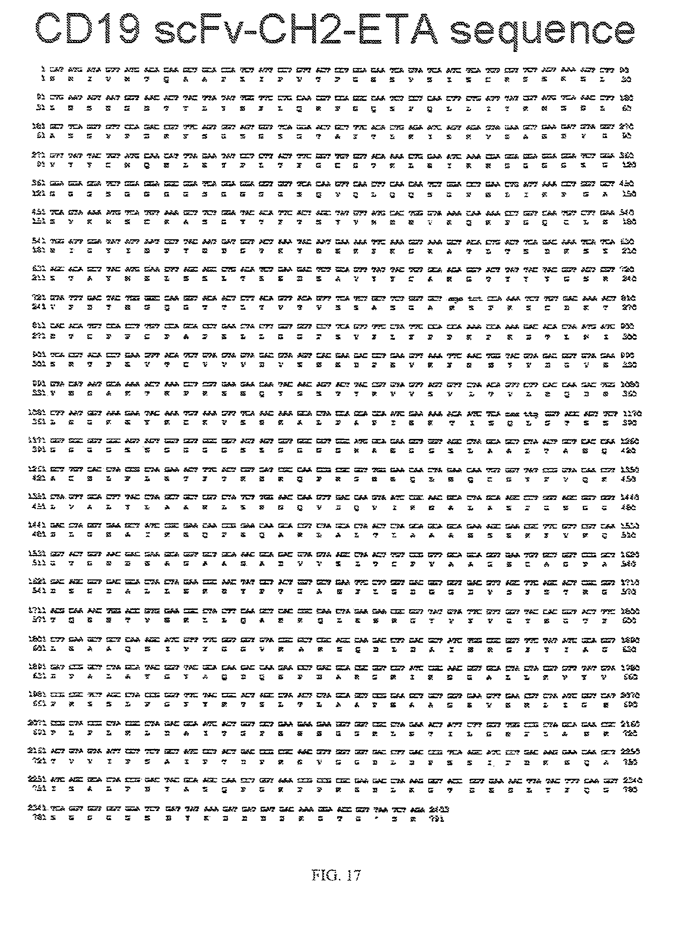

Figure 17 shows the nucleotide and amino acid sequences of the CD 19 scFv-CH2-ETA fusion protein (SEQ ID NOS:12 and 13, respectively). Amino acid residues 1 to 261 define the variable regions of the light chain, amino acid residues 262 to 381 define the CH2 constant domain, amino acid residues 382 to 772 define Exotoxin A, amino acid residues 773 to 780 define a TEV cleavage site, amino acid residues 781 to 786 define the flexible peptide linker, and amino acid residues 782 to 791 at the carboxy terminus define the FLAG epitope tag.

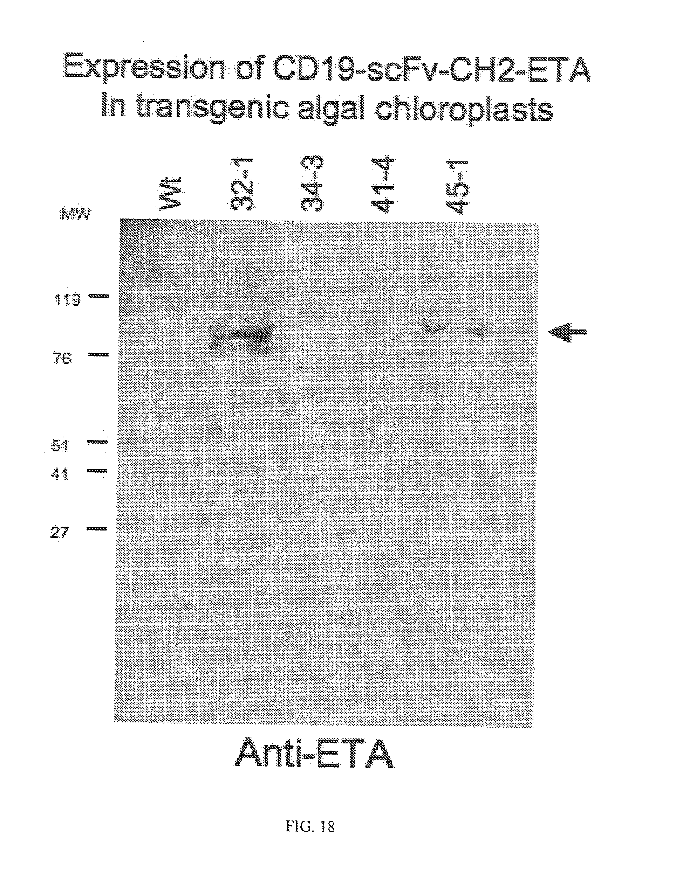

Figure 18 shows expression of an anti-CD19-scFv-heavy chain CH2 domain-exotoxin A chimeric protein. Four transgenic lines, 32-1, 34-3, 41-4 and 45-1 were analyzed by western blot analysis for the accumulation of the chimeric protein. Protein from non-transformed wild type cells (Wt) was loaded in Lane 1. The chimeric antibody-toxin protein (arrowhead) accumulates as a soluble protein at the correct molecular weight (85 kD) in at least three of the transgenic lines, 32-1, 41-4 and 45-1. The chimeric protein was visualized using an anti-ETA antibody.

### DETAILED DESCRIPTION OF THE INVENTION

It is to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

The present invention discloses recombinant proteins containing a genetic fusion between a first protein or peptide and a protein toxin or peptide toxin, where such a fusion protein is produced in a eukaryotic cell and would normally be lethal to such cells. The recombinant method does not require modifying the toxin or nucleic acid sequence encoding the toxin to alter toxin activity. In one embodiment, a disclosed fusion protein comprises an immunoglobulin binding domain, including but not limited to, an anti-CD19 single chain antibody (CD19) and a bacterial protein, including but not limited to, exotoxin A protein (ETA) of *Pseudomonas.* In one aspect, the a CD19-ETA fusion protein gene may be transformed into the chloroplast of a plant cell, including but not limited to, the green algae *C. reinhardtii* and a bioactive antibody-toxin may be produced in eukaryotic cell organelles (*e.g*., chloroplasts). In another aspect, the purified CD19-ETA is cytotoxic to CD 19 positive Ramos human cell line, as well as cytotoxic to activated peripheral blood *lymphocytes, in vitro.*

A plant derived protein toxin or peptide toxin, including but not limited to, gelonin or ricin.

Data are provided that shows that eukaryotic toxins can be expressed in eukaryotic cells if the toxin is produced within a subcellular organelle, like the chloroplast. These data also demonstrate the utility of plants, including but not limited to, green algae, for the production of complex multi-domain proteins as soluble bioactive therapeutic agents.

As used herein "cognate" is used in a comparative sense to refer to genetic elements that are typically associated with a specific reference gene. For example, for the Photosystem II (PSII) *gene psbA (i.e.,* a specific reference gene), cognate genetic elements would include, but are not limited to, a *psbA* promoter, *psbA* 5' UTR, and *psbA* 3' UTR. Contrapositively, "non-cognate" would refer to genetic elements that are not typically related to a specific reference gene. For example, but not limited to, where a chimeric construct comprising *a psbA* promoter and *psbD 5'* UTR is to be homologously recombined at *a psbA* site, the 5' UTR in the construct would be non-cognate to *psbA.*

As used herein "nucleic acid signaling element" is used broadly herein to refer to a nucleotide sequence that regulates the transcription or translation of a polynucleotide or the localization of a polypeptide to which it is operatively linked. A nucleic acid signaling element can be a promoter, enhancer, transcription terminator, an initiation (start) codon, a splicing signal for intron excision and maintenance of a correct reading frame, a STOP codon, an amber or ochre codon, an IRES, an RBS, a sequence encoding a protein intron (intein) acceptor or donor splice site, or a sequence that targets a polypeptide to a particular location, for example, a cell compartmentalization signal, which can be useful for targeting a polypeptide to the cytosol, nucleus, plasma membrane, endoplasmic reticulum, mitochondrial membrane or matrix, chloroplast membrane or lumen, medial trans-Golgi cisternae, or a lysosome or endosome. Cell compartmentalization domains are well known in the art and include, for example, a peptide containing amino acid residues 1 to 81 of human type II membrane-anchored protein galactosyltransferase, the chloroplast targeting domain from the nuclear-encoded small subunit of plant ribulose bisphosphate carboxylase, or amino acid residues 1 to 12 of the presequence of subunit IV of cytochrome c oxidase (see, also, Hancock et al., EMBO J 10:4033-4039, 1991; Buss et al., Mol. Cell. Biol. 8:3960-3963, 1988; U.S. Pat. No. 5,776,689). Inclusion of a cell compartmentalization domain in a polypeptide produced using a method of the invention can allow use of the polypeptide, which can comprise a protein complex, where it is desired to target the polypeptide to a particular cellular compartment in a cell.

As used herein "binding domain" means a region of a protein or peptide which allows for stereoselective, specific interaction with a cell surface markers, and includes, but is not limited to, antibodies, receptors, hormones, cytokines, chemokines, interferons, and fragments thereof.

As used herein "cell surface markers" means a polypeptide, carbohydrate, lipid or a combination thereof on the plasma surface of a cell. In one embodiment, such markers include clusters of differentiation (CD), including, but are not limited to, CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD18, CD19, CD20, CD21, CD22, CD23, Cd24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d, CD66e, and the like. In one embodiment, the CD is specific for B-cells. In a related aspect, the marker is CD 19.

As used herein "toxin" includes bacterial and plant derived toxins. For example, such toxins are proteins or peptides, and include botulism toxin, tetanus toxin, shigella neurotoxin, diphtheria toxin, hemolysins, leukocidins, anthrax toxin, adenylate cyclase toxin, cholera enterotoxin, *E. coli* LT toxin, *E. coli* ST toxin, exotoxins, shiga toxin, perfringens toxin, exotoxin A, pertussis toxin, toxic shock syndrome toxin, exfoliatin toxin, erythrogenic toxin, and the like. In one aspect, the toxin is endotoxin A. In another aspect, plant toxins include single chain ribosome inactivating proteins. In one aspect, proteinacious plant toxins are disclosed, including, but not limited to, gelonin and ricin. In another aspect, "obtained from a plant" means isolated, extracted or a polypeptide/peptide/protein which is normally expressed by a plant that is produced either synthetically or recombinantly.

As used herein "multifunctional" means having at least two functions. For example, a fusion protein comprising a binding domain and a toxin domain would be bifunctional.

As used herein "progeny" means a descendant or offspring, as a child, plant or animal. For example, daughter cells from a transgenic algae are progeny of the transgenic algae.

As used herein "transgene" means any gene carried by a vector or vehicle, where the vector or vehicle includes, but is not limited to, plasmids and viral vectors.

Integration of chimeric constructs into plastid genomes includes homologous recombination. Cells transformed by the methods of the present invention may be homoplasmic or heteroplasmic for the integration, wherein homoplastic means all copies of the transformed plastid genome carry the same chimeric construct.

As used herein, the term "modulate" refers to a qualitative or quantitative increase or decrease in the amount of an expressed gene product. For example, where the use of light increases or decreases the measured amount of protein or RNA expressed by a cell, such light modulates the expression of that protein or RNA. Modulation of expression includes autoregulation, where "autoregulation" refers to processes that maintain a generally constant physiological state in a cell or organism, and includes autorepression and autoinduction.

Autorepression is a process by which excess endogenous protein or endogenous mRNA results in decreasing the amount of expression of that endogenous protein. In a further related aspect, reduction of endogenous protein synthesis will result in increased transgene expression. Operatively linking non-cognate genetic elements (*e.g*., promoters) to the endogenous gene may be used to drive low levels of endogenous protein expression. Mutations may be introduced into the endogenous gene sequence and/or cognate genetic elements to reduce expression of the endogenous protein.

As used herein, the term "multiple cloning site" is used broadly to refer to any nucleotide or nucleotide sequence that facilitates linkage of a first polynucleotide to a second polynucleotide. Generally, a cloning site comprises one or a plurality of restriction endonuclease recognition sites, for example, a cloning site, or one or a plurality of recombinase recognition sites, for example, a loxP site or an att site, or a combination of such sites. The cloning site can be provided to facilitate insertion or linkage, which can be operative linkage, of the first and second polynucleotide, for example, a first polynucleotide encoding a first 5' UTR operatively linked to second polynucleotide comprising a homologous coding sequence encoding a polypeptide of interest, linked to a first 3' UTR, which is to be translated in a prokaryote or a chloroplast or both.

A chimeric construct is disclosed including a PSII reaction center protein gene promoter, PSII gene 5' UTR, a multiple cloning site (MCS), and a PSII gene 3' UTR, having the configuration:
PSII gene promoter-PSII gene 5' UTR-MCS-PSII gene 3' UTR.

The PSII gene UTRs may be from different PSII genes and may include, but are not limited to, a *psbD* 5' UTR and *a psbA* 5' UTR.

The PSII gene promoter may be *a psbA* or *psbD* promoter and the 3' UTR is a *psbA* 3' UTR.

The PSII gene promoter and PSII gene 5' UTR may be from *psbD,* or the PSII gene 3' UTR may be a *psbA* 3' UTR.

As used herein, the term "Photosystem II reaction center" refers to an intrinsic membrane-protein complex in the chloroplast made of D1 (*psbA* gene), D2 (*psbD* gene), alpha and beta subunits of cytochrome b-559 (*psbE* and *psbF* genes respectively), the *psbI* gene product and a few low molecular weight proteins *(e.g.,* 9 kDa peptide [*psbH* gene] and 6.5 kDa peptide [*psbW gene*]). Endogenous genes embrace chloroplast genes that exhibit autoregulation of translation, and include, but are not limited to, cytochrome *f* (*i.e.,* C-terminal domain) and photosystem I reaction center genes (*e.g., psaA, PsaB, PsaC, PsaJ*)*.*

As used herein, the term "operatively linked" means that two or more molecules are positioned with respect to each other such that they act as a single unit and effect a function attributable to one or both molecules or a combination thereof. For example, a polynucleotide encoding a polypeptide can be operatively linked to a transcriptional or translational regulatory element, in which case the element confers its regulatory effect on the polynucleotide similarly to the way in which the regulatory element would effect a polynucleotide sequence with which it normally is associated with in a cell.

The term "polynucleotide" or "nucleotide sequence" or "nucleic acid molecule" is used broadly herein to mean a sequence of two or more deoxyribonucleotides or ribonucleotides that are linked together by a phosphodiester bond. As such, the terms include RNA and DNA, which can be a gene or a portion thereof, a cDNA, a synthetic polydeoxyribonucleic acid sequence, or the like, and can be single stranded or double stranded, as well as a DNA/RNA hybrid. Furthermore, the terms as used herein include naturally occurring nucleic acid molecules, which can be isolated from a cell, as well as synthetic polynucleotides, which can be prepared, for example, by methods of chemical synthesis or by enzymatic methods such as by the polymerase chain reaction (PCR). It should be recognized that the different terms are used only for convenience of discussion so as to distinguish, for example, different components of a composition, except that the term "synthetic polynucleotide" as used herein refers to a polynucleotide that has been modified to reflect chloroplast codon usage.

In general, the nucleotides comprising a polynucleotide are naturally occurring deoxyribonucleotides, such as adenine, cytosine, guanine or thymine linked to 2'-deoxyribose, or ribonucleotides such as adenine, cytosine, guanine or uracil linked to ribose. Depending on the use, however, a polynucleotide also can contain nucleotide analogs, including non-naturally occurring synthetic nucleotides or modified naturally occurring nucleotides. Nucleotide analogs are well known in the art and commercially available, as are polynucleotides containing such nucleotide analogs. The covalent bond linking the nucleotides of a polynucleotide generally is a phosphodiester bond. However, depending on the purpose for which the polynucleotide is to be used, the covalent bond also can be any of numerous other bonds, including a thiodiester bond, a phosphorothioate bond, a peptide-like bond or any other bond known to those in the art as useful for linking nucleotides to produce synthetic polynucleotides.

A polynucleotide comprising naturally occurring nucleotides and phosphodiester bonds can be chemically synthesized or can be produced using recombinant DNA methods, using an appropriate polynucleotide as a template. In comparison, a polynucleotide comprising nucleotide analogs or covalent bonds other than phosphodiester bonds generally will be chemically synthesized, although an enzyme such as T7 polymerase can incorporate certain types of nucleotide analogs into a polynucleotide and, therefore, can be used to produce such a polynucleotide recombinantly from an appropriate template.

The term "recombinant nucleic acid molecule" is used herein to refer to a polynucleotide that is manipulated by human intervention. A recombinant nucleic acid molecule can contain two or more nucleotide sequences that are linked in a manner such that the product is not found in a cell in nature. In particular, the two or more nucleotide sequences can be operatively linked and, for example, can encode a fusion polypeptide, or can comprise an encoding nucleotide sequence and a regulatory element, particularly a PSII promoter operatively linked to a PSII 5' UTR. A recombinant nucleic acid molecule also can be based on, but manipulated so as to be different, from a naturally occurring polynucleotide, for example, a polynucleotide having one or more nucleotide changes such that a first codon, which normally is found in the polynucleotide, is biased for chloroplast codon usage, or such that a sequence of interest is introduced into the polynucleotide, for example, a restriction endonuclease recognition site or a splice site, a promoter, a DNA origin of replication, or the like.

One or more codons of an encoding polynucleotide can be biased to reflect chloroplast codon usage. Most amino acids are encoded by two or more different (degenerate) codons, and it is well recognized that various organisms utilize certain codons in preference to others. Such preferential codon usage, which also is utilized in chloroplasts, is referred to herein as "chloroplast codon usage". Table 1 (below) shows the chloroplast codon usage for C. *reinhardtii.*

**Table 1. Chloroplast Codon Usage for C. reinhardtii.**

| **Chloroplast Codon Usage in Chlamydomonas reinhardtii** | | | |
|---|---|---|---|
| **UUU 34.1*(348**)** | UCU 19.4(198) | UAU 23.7(242) | UGU 8.5(87) |
| **UUC 14.2(145)** | UCC 4.9(50) | UAC 10.4(106) | UGC 2.6(27) |
| **UUA 72.8(742)** | UCA 20.4(208) | UAA 2.7(28) | UGA 0.1(1) |
| **UUG 5.6(57)** | UCG 5.2(53) | UAG 0.7(7) | UGG 13.7(140) |
| **CUU 14.8(151)** | CCU 14.9(152) | CAU 11.1(113) | CGU 25.5(260) |
| **CUC 1.0(10)** | CCC 5.4(55) | CAC 8.4(86) | CGC 5.1(52) |
| **CUA 6.8(69)** | CCA 19.3(197) | CAA 34.8(355) | CGA 3.8(39) |
| **CUG 7.2(73)** | CCG 3.0(31) | CAG 5.4(55) | CGG 0.5(5) |
| **AUU 44.6(455)** | ACU 23.3(237) | AAU 44.0(449) | AGU 16.9(172) |
| **AUC 9.7(99)** | ACC 7.8(80) | AAC 19.7(201) | AGC 6.7(68) |
| **AUA 8.2(84)** | ACA 29.3(299) | AAA 61.5(627) | AGA 5.0(51) |
| **AUG 23.3(238)** | ACG 4.2(43) | AAG 11.0(112) | AGG 1.5(15) |
| **GUU 27.5(280)** | GCU 30.6(312) | GAU 23.8(243) | GGU 40.0(408) |
| **GUC 4.6(47)** | GCC 11.1(113) | GAC 11.6(118) | GGC 8.7(89) |
| **GUA 26.4(269)** | GCA 19.9(203) | GAA 40.3(411) | GGA 9.6(98) |
| **GUG 7.1(72)** | GCG 4.3(44) | GAG 6.9(70) | GGG 4.3(44) |

| | | | |
|---|---|---|---|
| * - Frequency of codon usage per 1,000 codons. ** - Number of times observed in 36 chloroplast coding sequences (10,193 codons). | | | |

The term "biased", when used in reference to a codon, means that the sequence of a codon in a polynucleotide has been changed such that the codon is one that is used preferentially in chloroplasts (see Table 1). A polynucleotide that is biased for chloroplast codon usage can be synthesized de novo, or can be genetically modified using routine recombinant DNA techniques, for example, by a site directed mutagenesis method, to change one or more codons such that they are biased for chloroplast codon usage. As disclosed herein, chloroplast codon bias can be variously skewed in different plants, including, for example, in alga chloroplasts as compared to tobacco.

Table 1 exemplifies codons that are preferentially used in alga chloroplast genes. The term "chloroplast codon usage" is used herein to refer to such codons, and is used in a comparative sense with respect to degenerate codons that encode the same amino acid but are less likely to be found as a codon in a chloroplast gene. The term "biased", when used in reference to chloroplast codon usage, refers to the manipulation of a polynucleotide such that one or more nucleotides of one or more codons is changed, resulting in a codon that is preferentially used in chloroplasts. Chloroplast codon bias is exemplified herein by the alga chloroplast codon bias as set forth in Table 1. The chloroplast codon bias can, but need not, be selected based on a particular plant in which a synthetic polynucleotide is to be expressed. The manipulation can be a change to a codon, for example, by a method such as site directed mutagenesis, by a method such as PCR using a primer that is mismatched for the nucleotide(s) to be changed such that the amplification product is biased to reflect chloroplast codon usage, or can be the de novo synthesis of polynucleotide sequence such that the change (bias) is introduced as a consequence of the synthesis procedure.

In addition to utilizing chloroplast codon bias as a means to provide efficient translation of a polypeptide, it will be recognized that an alternative means for obtaining efficient translation of a polypeptide in a chloroplast to re-engineer the chloroplast genome *(e.g.,* a *C. reinhardtii* chloroplast genome) for the expression of tRNAs not otherwise expressed in the chloroplast genome. Such an engineered algae expressing one or more heterologous tRNA molecules provides the advantage that it would obviate a requirement to modify every polynucleotide of interest that is to be introduced into and expressed from a chloroplast genome; instead, algae such as *C. reinhardtii* that comprise a genetically modified chloroplast genome can be provided and utilized for efficient translation of a polypeptide according to a method of the invention. Correlations between tRNA abundance and codon usage in highly expressed genes is well known in the art. In *E. coli,* for example, re-engineering of strains to express underutilized tRNAs has been shown to result in enhanced expression of genes which utilize these codons. Utilizing endogenous tRNA genes, site directed mutagenesis can be used to make a synthetic tRNA gene, which can be introduced into chloroplasts to complement rare or unused tRNA genes in a chloroplast genome such as a *C. reinhardtii* chloroplast genome.

Generally, the chloroplast codon bias selected for purposes of the present invention, including, for example, in preparing a synthetic polynucleotide as disclosed herein reflects chloroplast codon usage of a plant chloroplast, and includes a codon bias that, with respect to the third position of a codon, is skewed towards A/T, for example, where the third position has greater than about 66% AT bias, particularly greater than about 70% AT bias. As such, chloroplast codon biased for purposes of the present invention excludes the third position bias observed, for example, in *Nicotiana tabacus* (tobacco), shown to have 34.56% GC bias in the third codon position. In one embodiment, the chloroplast codon usage is biased to reflect alga chloroplast codon usage, for example, *C. reinhardtii,* which has about 74.6% AT bias in the third codon position.

In one embodiment, a method to produce functional fusion polypeptides/proteins is disclosed. The term "polypeptides/protein" is used broadly to refer to macromolecules comprising linear polymers of amino acids which act in biological systems, for example, as structural components, enzymes, chemical messengers, receptors, ligands, regulators, hormones, and the like. In one aspect, a plant cell or algae cell or progeny thereof is disclosed which contains a construct, where the construct includes, in operable linkage, nucleic acid signaling elements for homologous recombination and expression of the bifunctional fusion protein in a plant or algae plastid and a first polynucleotide sequence encoding a first polypeptide and a second polynucleotide sequence encoding a toxin, where the first and second polynucleotide sequences are expressed as a fusion protein. The fusion protein may include stabilizing molecules or domains, such as Fc domains and low complexity linkers. Such stabilizing molecules may form tripartite structures, which include a stabilizing domain-targeting domain-toxin domain. A fusion protein may comprise one or more stabilizing domains. Such tripartite molecules may also contain a small molecule drug, including, but not limited to therapeutic compounds. The tripartite molecule may comprise a purification domain (*e.g*., but not limited to, a His₆ (SEQ ID NO:14) or FLAG tag).

Such tripartite molecules may be encoded by a single polynucleotide. A functional binding domain of the tripartite molecule may comprise multimers of subunits to form a multimeric complex, where the tripartite structure is encoded with a first subunit of a multimer. The second or third or more subunits of the multimeric complex may be encoded on separate polynucleotides. The second, third or more subunits , may be are integrated into different sites in the chloroplast genome, where each integrated subunit encoding polynucleotide comprises separate recombinational targeting sequences, promoters/5' UTR regulatory sequences, and 3' UTR sequences. The multimeric complex may comprise a heavy chain and a light chain of an complete antibody.

In one embodiment, such fusion protein comprise multiple binding domains for targeting multiple surface markers. In one aspect, the fusion protein includes one or more binding domains which specifically target CD 19, CD20, and CD21. In other aspects, other clusters of differentiation (CD) may include, but are not limited to, CD1, CD2, CD3, CD4, CD5, CD6, CD7, CD8, CD9, CD10, CD11a, CD11b, CD11c, CDR11d, CDw12, CD13, CD14, CD15, CD15s, CD16, CDw17, CD18, CD22, CD23, Cd24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49aq, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d, CD66e, and the like.

In another aspect, such polypeptides/proteins would include functional protein complexes, such as antibodies. The term "antibody" is used broadly herein to refer to a polypeptide or a protein complex that can specifically bind an epitope of an antigen. As used in this invention, the term "epitope" refers to an antigenic determinant on an antigen, such as a cell surface marker, to which the paratope of an antibody, such as an CD 19 specific antibody, binds. Antigenic determinants usually consist of chemically active surface groupings of molecules, such as amino acids or sugar side chains, and can have specific three dimensional structural characteristics, as well as specific charge characteristics.

Generally, an antibody contains at least one antigen binding domain that is formed by an association of a heavy chain variable region domain and a light chain variable region domain, particularly the hypervariable regions. An antibody generated according to a method of the invention can be based on naturally occurring antibodies, for example, bivalent antibodies, which contain two antigen binding domains formed by first heavy and light chain variable regions and second heavy and light chain variable regions (*e.g*., an IgG or IgA isotype) or by a first heavy chain variable region and a second heavy chain variable region (V_{HH} antibodies), or on non-naturally occurring antibodies, including, for example, single chain antibodies, chimeric antibodies, bifunctional antibodies, and humanized antibodies, as well as antigen-binding fragments of an antibody, for example, an Fab fragment, an Fd fragment, an Fv fragment, and the like. A heterologous gene may encode a single chain antibody comprising a heavy chain operatively linked to a light chain.

Antigens that can be used in the present invention specific antibodies select polypeptides or polypeptide fragments. The polypeptide or peptide used to immunize an animal can be obtained by standard recombinant, chemical synthetic, or purification methods. As is well known in the art, in order to increase immunogenicity, an antigen can be conjugated to a carrier protein. Commonly used carriers include keyhole limpet hemocyanin (KLH), thyroglobulin, bovine serum albumin (BSA), and tetanus toxoid. The coupled peptide is then used to immunize the animal (*e.g*., a mouse, a rat, or a rabbit). In addition to such carriers, well known adjuvants can be administered with the antigen to facilitate induction of a strong immune response.

Polynucleotides useful for practicing a method of the producing such antibodies can be isolated from cells producing the antibodies of interest, for example, B cells from an immunized subject or from an individual exposed to a particular antigen, can be synthesized de novo using well known methods of polynucleotide synthesis, can be produced recombinantly or can be obtained, for example, by screening combinatorial libraries of polynucleotides that encode variable heavy chains and variable light chains and can be biased for chloroplast codon usage, if desired (see Table 1). These and other methods of making polynucleotides encoding, for example, chimeric, humanized, CDR-grafted, single chain, and bifunctional antibodies are well known to those skilled in the art.

Polynucleotides encoding humanized monoclonal antibodies, for example, can be obtained by transferring nucleotide sequences encoding mouse complementarity determining regions (CDRs) from heavy and light variable chains of the mouse immunoglobulin gene into a human variable domain gene, and then substituting human residues in the framework regions of the murine counterparts. General techniques for cloning murine immunoglobulin variable domains are known is the art, as well as methods for producing humanized monoclonal antibodies.

The disclosed methods can also be practiced using polynucleotides encoding human antibody fragments isolated from a combinatorial immunoglobulin library. Cloning and expression vectors that are useful for producing a human immunoglobulin phage library can be obtained, for example, from Stratagene Cloning Systems (La Jolla, Calif.).

A polynucleotide encoding a human monoclonal antibody also can be obtained, for example, from transgenic mice that have been engineered to produce specific human antibodies in response to antigenic challenge. In this technique, elements of the human heavy and light chain loci are introduced into strains of mice derived from embryonic stem cell lines that contain targeted disruptions of the endogenous heavy and light chain loci. The transgenic mice can synthesize human antibodies specific for human antigens, and the mice can be used to produce human antibody-secreting hybridomas, from which polynucleotides useful for practicing a method of the invention can be obtained. Methods for obtaining human antibodies from transgenic mice have been previously described, and such transgenic mice are commercially available (*e.g*., Abgenix, Inc.; Fremont Calif.).

Monoclonal antibodies used in the method disclosed herein are suited for use, for example, in immunoassays in which they can be utilized in liquid phase or bound to a solid phase carrier. In addition, the monoclonal antibodies in these immunoassays can be detectably labeled in various ways. Examples of types of immunoassays which can utilize monoclonal antibodies of the invention are competitive and non-competitive immunoassays in either a direct or indirect format. Examples of such immunoassays are the radioimmunoassay (RIA) and the sandwich (immunometric) assay. Detection of the antigens using the monoclonal antibodies of the invention can be done utilizing immunoassays which are run in either the forward, reverse, or simultaneous modes, including immunohistochemical assays on physiological samples. Those of skill in the art will know, or can readily discern, other immunoassay formats without undue experimentation.

The term "immunometric assay" or "sandwich immunoassay", includes simultaneous sandwich, forward sandwich and reverse sandwich immunoassays. These terms are well understood by those skilled in the art. Those of skill will also appreciate that antibodies according to the present invention will be useful in other variations and forms of assays which are presently known or which may be developed in the future.

Monoclonal antibodies produced by the method of the present invention may also be bound to many different carriers. Examples of well-known carriers include glass, polystyrene, polypropylene, polyethylene, dextran, nylon, amylases, natural and modified celluloses, polyacrylamides, agaroses and magnetite. The nature of the carrier can be either soluble or insoluble for purposes of the invention. Those skilled in the art will know of other suitable carriers for binding monoclonal antibodies, or will be able to ascertain such using routine experimentation.

In performing the assays it may be desirable to include certain "blockers" in the incubation medium (usually added with the labeled soluble antibody). The "blockers" are added to assure that non-specific proteins present in the experimental sample do not cross-link or destroy the antibodies on the solid phase support, or the radiolabeled indicator antibody, to yield false positive or false negative results. The selection of "blockers" therefore may add substantially to the specificity of the assays described in the present invention.

It has been found that a number of nonrelevant (*i.e*., nonspecific) antibodies of the same class or subclass (isotype) as those used in the assays (*e.g.,* IgG1, IgG2a, IgM, etc.) can be used as "blockers". The concentration of the "blockers" (normally 1-100 µg/µl) may be important, in order to maintain the proper sensitivity yet inhibit any unwanted interference by mutually occurring cross reactive proteins in the specimen.

In using a monoclonal antibody for the *in vivo* detection of antigen, the detectably labeled monoclonal antibody is given in a dose which is diagnostically effective. The term "diagnostically effective" means that the amount of detectably labeled monoclonal antibody is administered in sufficient quantity to enable detection of the site having the antigen of interest for which the monoclonal antibodies are specific. The concentration of detectably labeled monoclonal antibody which is administered should be sufficient such that the binding to those antigens/epitopes of interest is detectable compared to the background. Further, it is desirable that the detectably labeled monoclonal antibody be rapidly cleared from the circulatory system in order to give the best target-to-background signal ratio.

As a rule, the dosage of detectably labeled monoclonal antibody for *in vivo* diagnosis will vary depending on such factors as age, sex, and extent of disease of the individual. The dosage of monoclonal antibody can vary from about 0.001 mg/m² to about 500 mg/m², preferably 0.1 mg/m² to about 200 mg/m², most preferably about 0.1 mg/m² to about 10 mg/m². Such dosages may vary, for example, depending on whether multiple injections are given, tumor burden, and other factors known to those of skill in the art.

For *in vivo* diagnostic imaging, the type of detection instrument available is a major factor in selecting a given radioisotope. The radioisotope chosen must have a type of decay which is detectable for a given type of instrument. Still another important factor in selecting a radioisotope for *in vivo* diagnosis is that the half-life of the radioisotope be long enough so that it is still detectable at the time of maximum uptake by the target, but short enough so that deleterious radiation with respect to the host is minimized. Ideally, a radioisotope used *for in vivo* imaging will lack a particle emission, but produce a large number of photons in the 140-250 keV range, which may be readily detected by conventional gamma cameras.

For *in vivo* diagnosis, radioisotopes may be bound to immunoglobulin either directly or indirectly by using an intermediate functional group. Intermediate functional groups which often are used to bind radioisotopes which exist as metallic ions to immunoglobulins are the bifunctional chelating agents such as diethylenetriaminepentacetic acid (DTPA) and ethylenediaminetetraacetic acid (EDTA) and similar molecules. Typical examples of metallic ions which can be bound to the monoclonal antibodies of the invention are ¹¹¹In, ⁹⁷ Ru, ⁶⁷Ga, ⁶⁸Ga, ⁷²As, ⁸⁹Zr, and ²⁰¹Ti.

A monoclonal antibody produced by the method of the invention can also be labeled with a paramagnetic isotope for purposes of *in vivo* diagnosis, as in magnetic resonance imaging (MRI) or electron spin resonance (ESR). In general, any conventional method for visualizing diagnostic imaging can be utilized. Usually gamma and positron emitting radioisotopes are used for camera imaging and paramagnetic isotopes for MRI. Elements which are particularly useful in such techniques include ¹⁵⁷Gd, ⁵⁵Mn, ¹⁶²Dy, ⁵²Cr, and ⁵⁶Fe.

The polynucleotide also can be one encoding an antigen binding fragment of an antibody. Antigen binding antibody fragments, which include, for example, Fv, Fab, Fab', Fd, and F(ab')₂ fragments, are well known in the art, and were originally identified by proteolytic hydrolysis of antibodies. For example, antibody fragments can be obtained by pepsin or papain digestion of whole antibodies by conventional methods. Antibody fragments produced by enzymatic cleavage of antibodies with pepsin generate a 5S fragment denoted F(ab')₂. This fragment can be further cleaved using a thiol reducing agent and, optionally, a blocking group for the sulfhydryl groups resulting from cleavage of disulfide linkages, to produce 3.5S Fab' monovalent fragments. Alternatively, an enzymatic cleavage using pepsin may produce two monovalent Fab' fragments and an Fc fragment directly.

Another form of an antibody fragment is a peptide coding for a single complementarity-determining region (CDR). CDR peptides can be obtained by constructing a polynucleotide encoding the CDR of an antibody of interest, for example, by using the polymerase chain reaction to synthesize the variable region from RNA of antibody-producing cells. Polynucleotides encoding such antibody fragments, including subunits of such fragments and peptide linkers joining, for example, a heavy chain variable region and light chain variable region, can be prepared by chemical synthesis methods or using routine recombinant DNA methods, beginning with polynucleotides encoding full length heavy chains and light chains, which can be obtained as described above.

The antibodies produced by the present invention can also include single chain antibodies ("SCA"). These antibodies are genetically engineered single chain molecules containing the variable region of a light chain and the variable region of a heavy chain, linked by a suitable, flexible polypeptide linker.

As an alternative to full length antibodies, including monoclonal antibodies, an equally viable approach utilizes toxins fused to Fc regions (typically hinge, C_{H}2-C_{H}3 domains of heavy chain hIgG1, 2, 3, 4 or IgA, IgE, IgM or IgD molecules) of monoclonal antibodies. These Fc regions may be native, or modified in ways that increase or decrease their affinity with specific Fc receptors. For example, modifications to the Fc region of hIgG1 molecules can increase their interaction with FcγRIII on effector cells, thereby modulating ADCC. Likewise, modifications to Fc regions on hIgG1 can impact their interactions with FcγRIIB, the inhibitory Fc receptor, on effector cells, again to modulate ADCC or to kill a particular population of cells when fused to toxins of the present invention.

The Fc region allows antibodies to activate the immune system, and is selective/specific for antibody isotype. For example, in IgG, IgA and IgD antibody isotypes, the Fc region is composed of two identical protein fragments, derived from the second and third constant domains of the antibody's two heavy chains; IgM and IgE Fc regions contain three heavy chain constant domains (CH domains 2-4) in each polypeptide chain. In one aspect, the Fc region is hIgG1Fc.

The Fc portion of these molecules imparts increased half life to the toxins to which they are fused through their increased size and provides a standardized and potentially modifiable means of purification via Protein A or G affinity chromatography.

Another application of Fc fusion proteins as disclosed is for increasing the potency of the toxins which are fused to Fc regions. While not being bound by theory, this increase in potency may be conferred by several mechanisms, including, but not limited to, increasing molecular weight leading to oligomerization. Such oligomerization can result in decreased loss of the toxin via renal filtration. In one embodiment, a nucleic acid construct is disclosed including, in operable linkage, nucleic acid signaling elements for homologous recombination and expression of the fusion protein in a plant or algae plastid and a first polynucleotide sequence encoding a first polypeptide and a second polynucleotide sequence encoding a toxin.

In one aspect, the first polynucleotide encodes an Fc region, or fragment thereof, where the Fc region, is a protein that mediates different immuological effects including, but not limited to, opsonization, cell lysis, and degranulation of mast cells, basophils and eosinophils.

IgG exhibits the highest synthetic rate and longest biological half-life of any immunoglobulin in serum. Complement activation is possibly the most important biological function of IgG. Activation of the complement cascade by the classical pathway is initiated by binding of C 1 to sites on the Fc portion of human IgG. Another vital function of the human IgG is its ability to bind to cell surface Fc receptors. Once it is fixed to the surface of certain cell types, the IgG antibody can complex antigen and facilitate clearance of antigens or immune complexes by phagocytosis. Three classes of human IgG Fc receptors (FcR) on leukocytes have been reported: the FcR-I, FcR-II, and low affinity receptor [FcR-lo]. These are distinguished by their presence on different cell types, by their molecular weights and by their differential abilities to bind untreated or aggregated IgG myeloma protein of the four subclasses. These receptors are expressed differentially on overlapping populations of leukocytes: FcR-I on monocytes; FcR-II on monocytes neutrophils, eosinophils, platelets, and B cells; and FcR-lo on neutrophils, macrophages, and killer T cells.

In one embodiment, a nucleic acid construct is disclosed including, in operable linkage, nucleic acid signaling elements for homologous recombination and expression of the fusion protein in a plant or algae plastid and a first polynucleotide sequence encoding a polypeptide consisting essentially of an Fc region and a second polynucleotide sequence encoding a toxin.

In one aspect, a toxin is functional in a eukaryotic cell, and may include, but is not limited to, a cellular toxin such as single-chain bacterial toxins (*e.g., Pseudomonas* exotoxin, diphtheria toxin) or plant holotoxins (*e.g*., class II ribosome inactivating proteins such as ricin, abrin, mistletoe lectin, moceccin, or abrin) or hemitoxins (*e.g*., class I ribosome inactivating proteins such as gelonin, saporin, pokeweed antiviral protein, bouganin, or bryodin 1). In a related aspect, the toxin is exotoxin A. In another aspect, the toxin is a toxin derived from a plant, and includes, but is not limited to, gelonin.

Single celled alga, like *C*. *reinhardtii,* are essentially water borne plants and as such can produce proteins in a very cost effective manner. In addition, algae can be grown in complete containment, and there are a number of companies around the world that have develop large scale production of algae as human nutraceuticals or as a food source for farmed fish and other organisms. Capitalization costs for an algal production facility is also much less costly than for other types of cell culture, mainly because of the nature of algae and it's ability to grow with minimal input, using CO₂ as a carbon source and sunlight as an energy source. Although in many ways algae are an ideal system for therapeutic protein production there are a number of technical challenges that need to be met before algae can be used as an efficient production platform. Among these challenges are developing vectors that allow for consistent high levels of protein expression.

A recombinant nucleic acid molecule useful in a method of the invention can be contained in a vector. The vector can be any vector useful for introducing a polynucleotide into a chloroplast and, preferably, includes a nucleotide sequence of chloroplast genomic DNA that is sufficient to undergo homologous recombination with chloroplast genomic DNA, for example, a nucleotide sequence comprising about 400 to 1500 or more substantially contiguous nucleotides of chloroplast genomic DNA. A number of chloroplast vectors and methods for selecting regions of a chloroplast genome for use as a vector have been described.

The entire chloroplast genome of *C*. *reinhardtii* has been sequenced (Maul et al., Plant Cell (2002) 14(11):2659-79; GenBank Acc. No. BK000554). Generally, the nucleotide sequence of the chloroplast genomic DNA is selected such that it is not a portion of a gene, including a regulatory sequence or coding sequence, particularly a gene that, if disrupted due to the homologous recombination event, would produce a deleterious effect with respect to the chloroplast, for example, for replication of the chloroplast genome, or to a plant cell containing the chloroplast. In this respect, the Accession No. disclosing the *C*. *reinhardtii* chloroplast genome sequence also provides maps showing coding and non-coding regions of the chloroplast genome, thus facilitating selection of a sequence useful for constructing a vector of the invention. For example, the chloroplast vector, p322, which is used in experiments disclosed herein, is a clone extending from the *Eco* (*Eco RI*) site at about position 143.1 kb to the *Xho* (*Xho I*) site at about position 148.5 kb.

The vector also can contain any additional nucleotide sequences that facilitate use or manipulation of the vector, for example, one or more transcriptional regulatory elements, a sequence encoding a selectable markers, one or more cloning sites, and the like. In one embodiment, the chloroplast vector contains a prokaryote origin of replication (ori), for example, an *E. coli* ori, thus providing a shuttle vector that can be passaged and manipulated in a prokaryote host cell as well as in a chloroplast.

The methods of the present invention are exemplified using the microalga, *C*. *reinhardtii.* The use of microalgae to express a polypeptide or protein complex according to a method of the invention provides the advantage that large populations of the microalgae can be grown, including commercially (Cyanotech Corp.; Kailua-Kona HI), thus allowing for production and, if desired, isolation of large amounts of a desired product. However, the ability to express, for example, functional mammalian polypeptides, including protein complexes, in the chloroplasts of any plant allows for production of crops of such plants and, therefore, the ability to conveniently produce large amounts of the polypeptides.

In one embodiment, a method of expressing a chimeric gene is disclosed including transforming an algae cell by replacing an endogenous chloroplast gene via integration of a chimeric construct having a heterologous coding sequence, a promoter sequence, and at least one UTR, wherein the promoter is cognate or non-cognate to the endogenous chloroplast gene, and cultivating the transformed algae cell. A gene product encoded by the heterologous coding sequence may beconstitutively expressed. In a related aspect, the cells are homoplasmic for the integration.

A method of expressing a chimeric gene may include transforming an algae cell by replacing *psbA* via integration of a chimeric construct comprising a nucleic acid sequence encoding a fusion protein of the present invention, such as those set forth in SEQ ID NO:5, SEQ ID NO:7, SEQ ID NO:11 or SEQ ID NO:13, a promoter sequence, and one or more UTRs, where the promoter is cognate or non-cognate to the endogenous chloroplast gene, and cultivating the transformed algae cell. In one aspect, at least two UTRs *are psbA* and *psbD* UTRs. In a related aspect, the nucleic acid sequence (*e.g*., SEQ ID NO:4, SEQ ID NO:6, SEQ ID NO:10, or SEQ ID NO:12) is driven by *a psbA* or other promoter.

In one embodiment, an algae cell transformed by the methods of the invention is disclosed, where the algae includes, but is not limited to, *Chlamydomonas reinhardtii.*

Accordingly, the methods of the invention can be practiced using any plant having chloroplasts, including, for example, macroalgae, for example, marine algae and seaweeds, as well as plants that grow in soil, for example, corn (*Zea mays*), Brassica sp. (*e.g., B. napus, B. rapa, B. juncea*), particularly those Brassica species useful as sources of seed oil, alfalfa *(Medicago sativa),* rice (*Oryza sativa*), rye (*Secale cereale*), sorghum (*Sorghum bicolor, Sorghum vulgare*)*,* millet *(e.g.,* pearl millet (*Pennisetum glaucum*), proso millet (*Panicum miliaceum*), foxtail millet (*Setaria italica*)*,* finger millet *(Eleusine coracana)),* sunflower *(Helianthus annuus),* safflower *(Carthamus tinctorius),* wheat *(tritium aestivum),* soybean *(Glycine max),* tobacco *(Nicotiana tabacum),* potato *(Solanum tuberosum),* peanuts *(Arachis hypogaea),* cotton *(Gossypium barbadense, Gossypium hirsutum),* sweet potato *(Ipomoea batatus),* cassaya *(Manihot esculenta),* coffee *(Cofea spp.),* coconut *(Cocos nucifera),* pineapple *(Ananas comosus),* citrus trees *(Citrus spp.),* cocoa *(Theobroma cacao),* tea *(Camellia sinensis),* banana *(Musa spp.),* avocado *(Persea ultilane),* fig *(Ficus casica),* guava *(Psidium guajava),* mango *(Mangifera indica),* olive *(Olea europaea),* papaya *(Carica papaya),* cashew *(Anacardium occidentale),* macadamia *(Macadamia integrifolia),* almond *(Prunus amygdalus),* sugar beets *(Beta vulgaris),* sugarcane *(Saccharum spp.),* oats, duckweed (Lemna), barley, tomatoes *(Lycopersicon esculentum),* lettuce *(e.g., Lactuca sativa),* green beans *(Phaseolus vulgaris),* lima beans *(Phaseolus limensis),* peas *(Lathyrus spp.),* and members of the genus *Cucumis* such as cucumber (C. *sativus),* cantaloupe *(C. cantalupensis),* and musk melon (C. *melo).* Ornamentals such as azalea *(Rhododendron spp.),* hydrangea *(Macrophylla hydrangea),* hibiscus *(Hibiscus rosasanensis),* roses *(Rosa spp.),* tulips *(Tulipa spp.),* daffodils *(Narcissus spp.),* petunias *(Petunia hybrida),* carnation *(Dianthus caryophyllus),* poinsettia *(Euphorbia pulcherrima),* and chrysanthemum are also included. Additional ornamentals useful for practicing a method of the invention include impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia. Conifers that may be employed in practicing the present invention include, for example, pines such as loblolly pine *(Pinus taeda),* slash pine *(Pinus elliotii),* ponderosa pine *(Pinus ponderosa),* lodgepole pine *(Pinus contorta),* and Monterey pine *(Pinus radiata),* Douglas-fir *(Pseudotsuga menziesii);* Western hemlock *(Tsuga ultilane);* Sitka spruce *(Picea glauca);* redwood *(Sequoia sempervirens);* true firs such as silver fir *(Abies amabilis)* and balsam fir *(Abies balsamea);* and cedars such as Western red cedar *(Thuja plicata)* and Alaska yellow-cedar *(Chamaecyparis nootkatensis).*

Leguminous plants useful for practicing a method of the invention include beans and peas. Beans include guar, locust bean, fenugreek, soybean, garden beans, cowpea, mung bean, lima bean, fava bean, lentils, chickpea, etc. Legumes include, but are not limited to, Arachis, *e.g.,* peanuts, Vicia, *e.g.,* crown vetch, hairy vetch, adzuki bean, mung bean, and chickpea, Lupinus, *e.g.,* lupine, trifolium, Phaseolus, *e.g.,* common bean and lima bean, Pisum, *e.g.,* field bean, Melilotus, *e.g.,* clover, Medicago, *e.g.,* alfalfa, Lotus, *e.g.,* trefoil, lens, *e.g.,* lentil, and false indigo. Preferred forage and turf grass for use in the methods of the invention include alfalfa, orchard grass, tall fescue, perennial ryegrass, creeping bent grass, and redtop. Other plants useful in the invention include Acacia, aneth, artichoke, arugula, blackberry, canola, cilantro, clementines, escarole, eucalyptus, fennel, grapefruit, honey dew, jicama, kiwifruit, lemon, lime, mushroom, nut, okra, orange, parsley, persimmon, plantain, pomegranate, poplar, radiata pine, radicchio, Southern pine, sweetgum, tangerine, triticale, vine, yams, apple, pear, quince, cherry, apricot, melon, hemp, buckwheat, grape, raspberry, chenopodium, blueberry, nectarine, peach, plum, strawberry, watermelon, eggplant, pepper, cauliflower, Brassica, *e.g.,* broccoli, cabbage, ultilan sprouts, onion, carrot, leek, beet, broad bean, celery, radish, pumpkin, endive, gourd, garlic, snapbean, spinach, squash, turnip, ultilane, chicory, groundnut and zucchini.

A method disclosed herein can generate a plant containing chloroplasts that are genetically modified to contain a stably integrated polynucleotide. The integrated polynucleotide can comprise, for example, an encoding polynucleotide operatively linked to a first and second UTR as defined herein. A transgenic (transplastomic) plant, which comprises one or more chloroplasts containing a polynucleotide encoding one or more heterologous polypeptides, including polypeptides that can specifically associate to form a functional protein complex is disclosed.

The term "plant" is used broadly herein to refer to a eukaryotic organism containing plastids, particularly chloroplasts, and includes any such organism at any stage of development, or to part of a plant, including a plant cutting, a plant cell, a plant cell culture, a plant organ, a plant seed, and a plantlet. A plant cell is the structural and physiological unit of the plant, comprising a protoplast and a cell wall. A plant cell can be in the form of an isolated single cell or a cultured cell, or can be part of higher organized unit, for example, a plant tissue, plant organ, or plant. Thus, a plant cell can be a protoplast, a gamete producing cell, or a cell or collection of cells that can regenerate into a whole plant. As such, a seed, which comprises multiple plant cells and is capable of regenerating into a whole plant, is considered plant cell for purposes of this disclosure. A plant tissue or plant organ can be a seed, protoplast, callus, or any other groups of plant cells that is organized into a structural or functional unit. Particularly useful parts of a plant include harvestable parts and parts useful for propagation of progeny plants. A harvestable part of a plant can be any useful part of a plant, for example, flowers, pollen, seedlings, tubers, leaves, stems, fruit, seeds, roots, and the like. A part of a plant useful for propagation includes, for example, seeds, fruits, cuttings, seedlings, tubers, rootstocks, and the like.

A method of producing a heterologous polypeptide or protein complex in a chloroplast or in a transgenic plant can further include a step of isolating an expressed polypeptide or protein complex from the plant cell chloroplasts. As used herein, the term "isolated" or "substantially purified" means that a polypeptide or polynucleotide being referred to is in a form that is relatively free of proteins, nucleic acids, lipids, carbohydrates or other materials with which it is naturally associated. Generally, an isolated polypeptide (or polynucleotide) constitutes at least twenty percent of a sample, and usually constitutes at least about fifty percent of a sample, particularly at least about eighty percent of a sample, and more particularly about ninety percent or ninety-five percent or more of a sample.

An algae extract obtained from an algae cell transformed by replacing an endogenous chloroplast gene via integration of a chimeric construct having a heterologous coding sequence, a promoter sequence, and one or more UTRs, where the promoter is cognate or non-cognate to the endogenous chloroplast gene is disclosed.

The term "heterologous" is used herein in a comparative sense to indicate that a nucleotide sequence (or polypeptide) being referred to is from a source other than a reference source, or is linked to a second nucleotide sequence (or polypeptide) with which it is not normally associated, or is modified such that it is in a form that is not normally associated with a reference material. For example, a polynucleotide encoding an antibody is heterologous with respect to a nucleotide sequence of a plant chloroplast, as are the components of a recombinant nucleic acid molecule comprising, for example, a first nucleotide sequence operatively linked to a second nucleotide sequence, and is a polynucleotide introduced into a chloroplast where the polynucleotide is not normally found in the chloroplast.

The chloroplasts of higher plants and algae likely originated by an endosymbiotic incorporation of a photosynthetic prokaryote into a eukaryotic host. During the integration process genes were transferred from the chloroplast to the host nucleus. As such, proper photosynthetic function in the chloroplast requires both nuclear encoded proteins and plastid encoded proteins, as well as coordination of gene expression between the two genomes. Expression of nuclear and chloroplast encoded genes in plants is acutely coordinated in response to developmental and environmental factors.

In chloroplasts, regulation of gene expression generally occurs after transcription, and often during translation initiation. This regulation has been shown to be dependent upon the chloroplast translational apparatus, as well as nuclear-encoded regulatory factors. The chloroplast translational apparatus generally resembles that in bacteria; chloroplasts contain 70S ribosomes; have mRNAs that lack 5' caps and generally do not contain 3' poly-adenylated tails; and translation is inhibited in chloroplasts and in bacteria by selective agents such as chloramphenicol.

Several RNA elements that act as mediators of translational regulation have been identified within the 5'UTR's of chloroplast mRNAs. These elements may interact with nuclear-encoded factors and generally do not resemble known prokaryotic regulatory sequences.

A vector or other recombinant nucleic acid molecule of the invention can include a nucleotide sequence encoding a reporter polypeptide or other selectable marker. The term "reporter" or selectable marker" refers to a polynucleotide (or encoded polypeptide) that confers a detectable phenotype. A reporter generally encodes a detectable polypeptide, for example, a green fluorescent protein or an enzyme such as luciferase, which, when contacted with an appropriate agent (a particular wavelength of light or luciferin, respectively) generates a signal that can be detected by eye or using appropriate instrumentation. A selectable marker generally is a molecule that, when present or expressed in a cell, provides a selective advantage (or disadvantage) to the cell containing the marker, for example, the ability to grow in the presence of an agent that otherwise would kill the cell.

A selectable marker can provide a means to obtain prokaryotic cells or plant cells or both that express the marker and, therefore, can be useful as a component of a vector of the invention. Examples of selectable markers include those that confer antimetabolite resistance, for example, dihydrofolate reductase, which confers resistance to methotrexate; neomycin phosphotransferase, which confers resistance to the aminoglycosides neomycin, kanamycin and paromycin; hygro, which confers resistance to hygromycin; trpB, which allows cells to utilize indole in place of tryptophan; hisD, which allows cells to utilize histinol in place of histidine; mannose-6-phosphate isomerase which allows cells to utilize mannose; ornithine decarboxylase, which confers resistance to the ornithine decarboxylase inhibitor, 2-(difluoromethyl)-DL-ornithine; and deaminase from *Aspergillus terreus,* which confers resistance to Blasticidin S. Additional selectable markers include those that confer herbicide resistance, for example, phosphinothricin acetyltransferase gene, which confers resistance to phosphinothricin, a mutant EPSP-synthase, which confers glyphosate resistance, a mutant acetolactate synthase, which confers imidazolione or sulfonylurea resistance, a mutant *psbA,* which confers resistance to atrazine, or a mutant protoporphyrinogen oxidase, or other markers conferring resistance to an herbicide such as glufosinate. Selectable markers include polynucleotides that confer dihydrofolate reductase (DHFR) or neomycin resistance for eukaryotic cells and tetracycline; ampicillin resistance for prokaryotes such as *E. coli;* and bleomycin, gentamycin, glyphosate, hygromycin, kanamycin, methotrexate, phleomycin, phosphinotricin, spectinomycin, streptomycin, sulfonamide and sulfonylurea resistance in plants. Since a composition or a method of the invention can result in expression of a polypeptide in chloroplasts, it can be useful if a polypeptide conferring a selective advantage to a plant cell is operatively linked to a nucleotide sequence encoding a cellular localization motif such that the polypeptide is translocated to the cytosol, nucleus, or other subcellular organelle where, for example, a toxic effect due to the selectable marker is manifest.

The ability to passage a shuttle vector of the invention in a prokaryote allows for conveniently manipulating the vector. For example, a reaction mixture containing the vector and putative inserted polynucleotides of interest can be transformed into prokaryote host cells such as *E. coli,* amplified and collected using routine methods, and examined to identify vectors containing an insert or construct of interest. If desired, the vector can be further manipulated, for example, by performing site directed mutagenesis of the inserted polynucleotide, then again amplifying and selecting vectors having a mutated polynucleotide of interest. The shuttle vector then can be introduced into plant cell chloroplasts, wherein a polypeptide of interest can be expressed and, if desired, isolated according to a method of the invention.

A polynucleotide or recombinant nucleic acid molecule of the invention, which can be contained in a vector, including a vector of the invention, can be introduced into plant chloroplasts using any method known in the art. As used herein, the term "introducing" means transferring a polynucleotide into a cell, including a prokaryote or a plant cell, particularly a plant cell plastid. A polynucleotide can be introduced into a cell by a variety of methods, which are well known in the art and selected, in part, based on the particular host cell. For example, the polynucleotide can be introduced into a plant cell using a direct gene transfer method such as electroporation or microprojectile mediated (biolistic) transformation using a particle gun, or the "glass bead method", vortexing in the presence of DNA-coated microfibers or by liposome-mediated transformation, transformation using wounded or enzyme-degraded immature embryos.

Plastid transformation is a routine and well known method for introducing a polynucleotide into a plant cell chloroplast. Chloroplast transformation involves introducing regions of chloroplast DNA flanking a desired nucleotide sequence into a suitable target tissue; using, for example, a biolistic or protoplast transformation method (*e.g*., calcium chloride or PEG mediated transformation). Fifty bp to 3 kb flanking nucleotide sequences of chloroplast genomic DNA allow homologous recombination of the vector with the chloroplast genome, and allow the replacement or modification of specific regions of the plastome. Using this method, point mutations in the chloroplast 16S rRNA and rps12 genes, which confer resistance to spectinomycin or streptomycin, can be utilized as selectable markers for transformation, and can result in stable homoplasmic transformants, at a frequency of approximately one per 100 bombardments of target tissues. The presence of cloning sites between these markers provides a convenient nucleotide sequence for making a chloroplast vector, including a vector of the invention. Substantial increases in transformation frequency are obtained by replacement of the recessive rRNA or r-protein antibiotic resistance genes with a dominant selectable marker, the bacterial aadA gene encoding the spectinomycin-detoxifying enzyme aminoglycoside-3'-adenyltransferase. Approximately 15 to 20 cell division cycles following transformation are generally required to reach a homoplastidic state. Plastid expression, in which genes are inserted by homologous recombination into all of the up to several thousand copies of the circular plastid genome present in each plant cell, takes advantage of the enormous copy number advantage over nuclear-expressed genes to permit expression levels that can readily exceed 10% of the total soluble plant protein.

Known direct gene transfer methods, such as electroporation, also can be used to introduce a polynucleotide of the invention into a plant protoplast. Electrical impulses of high field strength reversibly permeabilize membranes allowing the introduction of the polynucleotide. Known methods of microinjection may also be performed. A transformed plant cell containing the introduced polynucleotide can be identified by detecting a phenotype due to the introduced polynucleotide, for example, expression of a reporter gene or a selectable marker.

Microprojectile mediated transformation also can be used to introduce a polynucleotide into a plant cell chloroplast. This method utilizes microprojectiles such as gold or tungsten, which are coated with the desired polynucleotide by precipitation with calcium chloride, spermidine or polyethylene glycol. The microprojectile particles are accelerated at high speed into a plant tissue using a device such as the BIOLISTIC PD-1000™ particle gun (BioRad; Hercules Calif.). Methods for the transformation using biolistic methods are well known. Microprojectile mediated transformation has been used, for example, to generate a variety of transgenic plant species, including cotton, tobacco, corn, hybrid poplar and papaya. Important cereal crops such as wheat, oat, barley, sorghum and rice also have been transformed using microprojectile mediated delivery. The transformation of most dicotyledonous plants is possible with the methods described above. Transformation of monocotyledonous plants also can be transformed using, for example, biolistic methods as described above, protoplast transformation, electroporation of partially permeabilized cells, introduction of DNA using glass fibers, the glass bead agitation method, and the like.

Reporter genes have been successfully used in chloroplasts of higher plants, and high levels of recombinant protein expression have been reported. In addition, reporter genes have been used in the chloroplast of C. *reinhardtii,* but, in most cases very low amounts of protein were produced. Reporter genes greatly enhance the ability to monitor gene expression in a number of biological organisms. In chloroplasts of higher plants, beta-glucuronidase (uidA), neomycin phosphotransferase (nptII), adenosyl-3-adenyltransf-erase (aadA), and the Aequorea victoria GFP have been used as reporter genes. Each of these genes has attributes that make them useful reporters of chloroplast gene expression, such as ease of analysis, sensitivity, or the ability to examine expression in situ.

Effective concentrations of the compositions described herein or pharmaceutically acceptable salts or other derivatives thereof are mixed with a suitable pharmaceutical carrier or vehicle. Derivatives of the compounds, such as salts of the compounds or prodrugs of the compounds may also be used in formulating effective pharmaceutical compositions. The concentrations of the compounds are effective for delivery of an amount, upon administration, that ameliorates the symptoms of the disease. Typically, the compositions are formulated for single dosage administration.

Upon mixing or addition of the compound(s), the resulting mixture may be a solution, suspension, emulsion or the like. The form of the resulting mixture depends upon a number of factors, including the intended mode of administration and the solubility of the compound in the selected carrier or vehicle. The effective concentration is sufficient for ameliorating the symptoms of the disease, disorder or condition treated and may be empirically determined.

Pharmaceutical carriers or vehicles suitable for administration of the compounds described herein include any such carriers known to those skilled in the art to be suitable for the particular mode of administration. In addition, the compounds may be formulated as the sole pharmaceutically active ingredient in the composition or may be combined with other active ingredients.

The active compounds can be administered by any appropriate route, for example, orally, parenterally, intravenously, intradermally, subcutaneously, or topically, in liquid, semi-liquid or solid form and are formulated in a manner suitable for each route of administration. Preferred modes of administration include oral and parenteral modes of administration. The active compound is included in the pharmaceutically acceptable carrier in an amount sufficient to exert a therapeutically useful effect in the absence of undesirable side effects on the patient treated. In one aspect, treated may be performed by contacting cells with the fusion protein of the invention *ex vivo.*

The therapeutically effective concentration may be determined empirically by testing the compounds in known *in vitro* and *in vivo* systems as described herein or known to those of skill in this art and then extrapolated therefrom for dosages for humans.

The concentration of active compound in the drug composition will depend on absorption, inactivation and excretion rates of the active compound, the dosage schedule, and amount administered as well as other factors known to those of skill in the art.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

A fusion protein as set forth in SEQ ID NO:5, SEQ ID NO:7, SEQ ID NO:11 and SEQ ID NO:13 is disclosed, including fusion protein-containing compositions admixed with pharmaceutically acceptable carriers. Such fusion protein compositions can be used to treat a subject with a proliferative cell disorder, including B-cell derived proliferative disorders. Such a proliferative disorder includes, but is not limited to, neoplasias, such as B-cell lymphomas.

A composition may include the fusion protein in combination with chemotherapeutic compounds, where such a combination may be used to treat a subject in need thereof. Such chemotherapeutics include, but are not limited to, Aclacinomycins, Actinomycins, Adriamycins, Ancitabines, Anthramycins, Azacitidines, Azaserines, 6-Azauridines, Bisantrenes, Bleomycins, Cactinomycins, Carmofurs, Carmustines, Carubicins, Carzinophilins, Chromomycins, Cisplatins, Cladribines, Cytarabines, Dactinomycins, Daunorubicins, Denopterins, 6-Diazo-5-Oxo-L-Norleucines, Doxifluridines, Doxorubicins, Edatrexates, Emitefurs, Enocitabines, Fepirubicins, Fludarabines, Fluorouracils, Gemcitabines, Idarubicins, Loxuridines, Menogarils, 6-Mercaptopurines, Methotrexates, Mithramycins, Mitomycins, Mycophenolic Acids, Nogalamycins, Olivomycines, Peplomycins, Pirarubicins, Piritrexims, Plicamycins, Porfiromycins, Pteropterins, Puromycins, Retinoic Acids, Streptonigrins, Streptozocins, Tagafurs, Tamoxifens, Thiamiprines, Thioguanines, Triamcinolones, Trimetrexates, Tubercidins, Vinblastines, Vincristines, Zinostatins, and Zorubicins.

The tablets, pills, capsules, troches and the like can contain any of the following ingredients, or compounds of a similar nature: a binder, such as microcrystalline cellulose, gum tragacanth and gelatin; an excipient such as starch and lactose, a disintegrating agent such as, but not limited to, alginic acid and corn starch; a lubricant such as, but not limited to, magnesium stearate; a glidant, such as, but not limited to, colloidal silicon dioxide; a sweetening agent such as sucrose or saccharin; and a flavoring agent such as peppermint, methyl salicylate, and fruit flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The compounds can also be administered as a component of an elixir, suspension, syrup, wafer, chewing gum or the like. A syrup may contain, in addition to the active compounds, sucrose as a sweetening agent and certain preservatives, dyes and colorings and flavors. The active materials can also be mixed with other active materials which do not impair the desired action, or with materials that supplement the desired action.

The following examples are provided to further illustrate the embodiments of the present invention, but are not intended to limit the scope of the invention. While they are typical of those that might be used, other procedures, methodologies, or techniques known to those skilled in the art may alternatively be used.

### EXAMPLE I

### EXPERIMENTAL PROTOCOLS AND METHODS FOR GENERATION OF ANTIBODY-TOXIN FUSIONS

*Synthesis of antibody and toxin genes, and construction of antibody-toxin fusion proteins.* Coding regions for all recombinant proteins were synthesized *de novo* in *C. reinhardtii* chloroplast condon bias (Franklin et al. Plant J (2002) 30:733-744, Mayfield et al., Proc Natl Acad Sci USA (2003) 100:438-442, Mayfield et al., Plant J (2004) 37:449-458) using PCR based oligonucleotide gene assembly (Stemmer et al., Gene (1995) 164:49-53). The coding regions synthesized include anti-human CD19 scFv (Figure 1) antibody fragment (Meeker et al., Hybridoma (1984) 3:305-320), and domains II and III (Figure 2) of *Pseudomonas aeruginosa* exotoxin A (Li et al., Proc Natl Acad Sci USA (1995) 92:9308-9312). The 5' and 3' terminal primers used in these assemblies contained restriction sites for *Nde I, Xba I,* respectively, for ease in subsequent cloning. A FLAG epitope tag was placed at the carboxy terminus of each protein, for analysis of protein expression and for subsequent purification using anti-flag affinity resin (Sigma, St. Louis, MO).

A CD19-ETA antibody-toxin fusion protein was generated by linking the CD19 scFv to ETA domains II and III using an inframe serine glycine amino acid linker (low complexity) located between the carboxy terminus of the antibody fragment and amino terminus of the toxin (Figure 3). This fusion created a 2022-bp gene expressing a single polypeptide of 662 amino acids.

*C. Reinhardtii transformation and growth conditions.* For expression of the CD 19 scFv, the *atpA* promoter and 5' UTR and the *rbcL* 3' UTR were used. For expression of ETA and CD19-ETA fusion *the psbA* promoter and 5' UTR, and psbA 3' UTR were used. Each of these promoters and UTRs were generated as previously described (Barnes et al., Mol Genet Genomics (2005) 274:625-636). The CD19 scFv expression cassette was placed in the Bam-HI site of integration plasmid p322 (Franklin et al., 2002), while the ETA and CD19-ETA expression cassettes contained flanking genomic sequences of the *psbA* gene that allowed for homologous recombination into the *C. reinhardtii* chloroplast genome as a replacement of the endogenous psbA gene (Manuell et al., Plant Biotech J (2007)).

*C. reinhardtii* strain 137c was grown in TAP medium (Gorman and Levine, Proc Natl Acad Sci USA (1965) 54:1665-1669) containing 1 mM 5-Fluorodeoxyuridine (FUDR) to late log phase under illumination of 4000 lux. Cells were pelleted by centrifugation and resuspended in TAP medium and 0.5 x 10⁸ cells were plated on agar plates containing TAP medium with 150 mg/L spectinomycin. The ETA, CD 19, and CD19-ETA expression cassettes were transformed separately into 137c cells along with the spectinomycin resistance ribosomal gene of plasmid p228 (Chlamydomonas Stock Center, Duke University). Colonies that grew on spectinomycin plates were screened by Southern blot for the presence of the CD19 scFv or ETA sequences, and transformants positive for the correct gene were taken through additional rounds of selection on specinomycin plates in order to obtain transformants that were homoplastic for each gene.

*Southern and Northern blots.* Southern blots and ³²P labeling of DNA for use as probes were carried out as described in Sambrook et al. (Molecular Cloning: A laboratory Manual, (1989), Cold Spring Harbor Laboratory Press, Cold Spring Harbor, NY), and Cohen et al. (Methods Enzymol (1998) 297:192-208). Genomic DNA from wt and the three transgenic lines was digested with restriction endonucleases, separated on agarose gels, and blotted to nylon membrane prior to being hybridized with ³²P-labeled probes. The probes for Southern and Northern blots included a 2.0 kb *BamHI*/*XhoI* fragment from the 3'end *of the psbA* locus, a 1182-bp coding region from ETA, a 674-bp coding region from CD19 scFv and a 600 bp fragment of the *psbA* cDNA. Northern and Southern blots were visualized utilizing a Packard Cyclone Storage Phosphor System^{™} equipped with Optiquant^{™} software.

*Recombinant protein expression and characterization. C. reinhardtii* proteins were isolated using a lysis buffer containing 10 mM Tris, 600 mM NaCl, 15% sucrose and 1 mM PMSF. To each gram of cell pellet 10 ml of lysis buffer was added and the cell ruptured by sonication. The insoluble and soluble phases were separated by centrifugation at 25,000 x g. Protein concentration was determined by Lowry protein assays. Proteins were separated by SDS-PAGE and blotted to a nitrocellulose membrane. Individual proteins were identified using antisera for ETA (Sigma, St. Louis, MO), or mouse anti-M2 Flag antibody (Sigma, St. Louis, MO). After washing with TBST the membranes were decorated with either a goat anti-rabbit antibody for ETA (Southern Biotech, Birmingham, AL), or a goat anti-mouse antibody for anti-flag (Southern Biotech, Birmingham, AL), both secondary antibodies were conjugated with alkaline phosphatase. The decorated proteins were visualized using alkaline phosphate assay.

For purification of the proteins, crude soluble extracts were incubated with M2 Flag resin following the method as described (Sigma, St. Louis, MO). The immuno-affinity purified proteins were eluted from the matrix with 100 mM Glycine and 600 mM NaCl pH 3.5 and then dialyzed against phosphate buffered saline (137 mM NaCl, 2.7 mM KCl, 1.8 mM K₂HPO₄, 10 mM Na₂HPO₄, pH 7.4). Purified proteins were used for bio-activity assays as described below.

*In vitro toxin activity assays.* To test for bio-activity of the exotoxin A protein, an *in vitro* ADP-ribosyltransferase assay was performed. The bio-activty of exotoxin A results from the catalytic transfer of ADP-ribose from NAD⁺ to eukaryotic elongation factor 2 (eEF2). The reaction mixture contained 20 mM tris pH 8.2, 50 µg/ml BSA, 1 mM EDTA, 1 mM DTT, 1 ng/µl eEF2 (wheat germ, Karen Browning UT, Austin), and 1.2 µl NAD⁺ mixture (1 µl ³²P NAD⁺ 800 ci/mmol added to 28.4 µl of NAD⁺ 40 mg/ml stock and the volume brought up to 800 µl with water) per 10 µl reaction. Fifty ng of purified ETA or purified CD19-ETA was added to each reaction. As negative controls, 50 ng of purified CD 19 alone, or 50 ng of crude *C. reinhardtii* soluble proteins, were used. The reaction mix was incubated 10 minutes at room temperature before a 1 X volume of protein loading dye was added and the proteins separated by SDS-PAGE. Following separation, the gel was dried and placed on a phosphorImager screen and viewed using a Packard Cyclone PhosphorImager System^{™} using Optiquant^{™} software.

*Isolation of peripheral blood lymphocytes from whole blood of normal donors.* Initially, 15 mls of whole blood was mixed with 15 mls of phosphate buffered saline (PBS) and underplayed with 10 ml of Ficoll Hypaque in a Falcon conical tube before centrifugation at 1750 rpm for 25 min. After centrifugation the layer containing the PBLs was removed and washed twice with PBS. A cell count was performed and the percentage of B cells in the PBL mixture was determined by FACS flow cytometry using CD20 or CD 19 antibody hybridization.

*Activation of B cells in PBL.* Cells were resuspended in RPMI culture media with HEPES and PSG and 5 x 10⁵- 1 x 10⁶ cells were added to each well of a 96 well plate. Twenty µl of anti-CD40 antibody at a concentration of 1 µg/ml was added to each well. The plate was then incubated at 4°C for 1-2 hours. Following the incubation at 4°C, an IgG anti-STAR81 was added to each well to final concentration of 0.2 µg/ml. The plate was then incubated at 37°C for an additional 1-2 hours. After the final incubation, 20 µl of IL2 and IL10 were added from a stock at 100 ng/ml to complete the activation of PBLs.

*CD19 antibody binding to Ramos and PBL cells.* 1 x 10⁶ cultured Ramos B cells or activated PBL were contacted with increasing amounts of CD19-ETA for one hour in FACS buffer (1 x PBS, 2% FCS and 0.05% sodium azide) at 4°C. CD19-ETA concentrations from 0.2 µg to 2.5 µg were used for binding assays and from 0.2 µg to 7.5 µg were used for cell killing assays. As controls, a commercially supplied anti-CD19 antibody conjugated with PE (Southern Biotech, Birmingham, AL) was used to verify that the target antigen, CD 19, was located on the cell surface of both cell types. B cell line Molt 4, which is CD19 negative, was used as a negative control to confirm that the CD19-ETA fusion protein was not binding nonspecifically to the B cells. After 1 hour incubation with CD19-ETA, the cells were washed three times with FACS buffer and then incubated with an anti-Flag antibody conjugated with FITC for 45 mins. After the incubation, cells were washed an additional three times and resuspended in 500 µl of FACS buffer and analyzed by FACS flow cytometry.

*Ramos and PBL cell killing assay.* Both Ramos cells and activated PBL were added to a 96 well plate at 1 x 10⁶ cells/well and cultured for 24 hours. The cells are then treated with varying amounts of CD19-ETA. As negative controls, the cells were also treated with CD 19 scFv or with exotoxin A, both expressed and purified from C. *reinhardtii.* Following 24 hours of incubation the cells were stained with annexin-5 antibody conjugated with FITC, or with propidium iodine (PI) and analyzed by FACS flow cytometry. Cell killing associated with apoptosis results in increased annexin-5 staining.

### EXAMPLE II

### SYNTHESIS AND ASSEMBLY OF AN ANTIBODY-TOXIN FUSION AND CONSTRUCTION OF CHLOROPLAST EXPRESSION VECTORS

In order to obtain high levels of protein expression in algal chloroplasts, transgene codons need to be optimized to reflect abundantly expressed genes of the C. *reinhardtii* chloroplast (Franklin et al., 2002; Mayfield et al., 2003; Mayfield and Schultz, 2004). Two recombinant protein codon regions were designed, a single chain antibody fragment that binds to CD19 protein found on human B cells (Meeker et al., 1984), and a truncated exotoxin A protein from *Pseudomonas aeruginosa* (Li et al., 1995) that lacks the cell binding domain, but retains the translocation and catalytic domains of the toxin. The amino acid sequences of the original proteins were maintained, but the codon usage was changed to reflect that of highly expressed C. *reinhardtii* chloroplast genes. The resulting chloroplast-optimized CD19 scFv coding sequence (CD19, Figure 1) was cloned into an expression cassette that contained the *atpA* promoter and 5' UTR and the *rbcL* 3' UTR, in the p322 expression cassette (Franklin et al., 2002). This cassette allows for transgene integration by homologous recombination between *the psbA* gene and the 16S rRNA gene in the inverted repeat of the chloroplast genome. The truncated exotoxin A protein (domains II and III) coding region (ETA, Figure 2) was cloned downstream *of the psbA* promoter and 5' UTR and upstream *of the psbA* 3' UTR (Manuell et al., 2007). The genomic sequences flanking the *psbA* 5' and 3' UTRs were also included to facilitate homologous replacement of the endogenous *psbA* gene (Manuell et al., 2007).

For assembly of the antibody toxin fusion, an inframe Kpn I restriction site was placed at the carboxy end of the CD19 scFv, and a corresponding inframe Kpn I site, along with a flexible amino acid linker, was placed at the amino terminus of the exotoxin A domain II gene. Ligation of these two fragments resulted in a fusion protein containing the CD 19 scFv as the amino half of the protein and exotoxin A domain II and III as the carboxy half of the protein (CD 19-ETA, Figure 3). The Kpn I site was subsequently removed by site directed mutagenesis. The CD19-ETA gene was ligated into the same psbA vector as the ETA gene to allow for integration into the chloroplast genome as a replacement *of the psbA* gene.

### EXAMPLE III

### INTRODUCTION OF THE RECOMBINANT GENES INTO THE C. REINHARDTII CHLOROPLAST GENOME

The chimeric CD 19, ETA, and CD19-ETA genes were introduced into the *C. reinhardtii* chloroplast genome by particle bombardment along with a selectable marker gene conferring spectinomycin resistance (Franklin et al., 2002). Spectinomycin resistant transformants were screened for the presence of the transgenes by Southern blot analysis. Chloroplasts contain multiple copies of their genome and several rounds of selection are required to achieve a homoplasmic strain with all copies of the organelle genome uniformly transformed. Using probes to both the coding regions of CD 19, ETA, or a flanking genome region, Southern blot analysis identified homoplastic lines for each of the three recombinant proteins (Figure 4). Hybridization of the blots with an ETA coding region probe identified a 1.6 kb band in ETA strain 1-4 and a 2.5 kb band in CD19-ETA strain 2-11, while hybridizing with a CD19 coding region probe identified a 2.5 kb band in the CD19-ETA strain 2-11 and a 1.3 kb band in the CD19 strain. Neither the CD19 or ETA genes were detected in the wt strain. Hybridization with a probe from the 3' end *of the psbA* locus yielded the expected 2.0 kb band in all samples.

### EXAMPLE IV

### ACCUMULATION OF RECOMBINANT mRNAS IN TRANSGENIC STRAINS

Northern blot analysis of total RNA was used to determine if the recombinant genes were transcribed correctly in transgenic *C. reinhardtii* chloroplasts. Ten µg of total RNA, isolated from wt and the three transgenic lines, was separated on denaturing agarose gels and blotted to nylon membrane. Duplicate filters were stained with ethidium bromide (Figure 5, left panel), or hybridized with a ³²P labeled *psbA* cDNA (Figure 5, central panel) a CD 19 coding region probe (Figure 5, central panel), or an ETA coding region probe (Figure 5, right panel). Each of the strains accumulated equal amounts of total RNA (stained bands), demonstrating that equal amounts of RNA were loaded for each lane, and that chloroplast transcription and mRNA accumulation are normal in the transgenic lines. The ETA probe identified an mRNA of approximately 2.2 kb in the ETA transgenic lane, and 3.1 kb in the CD19-ETA lane, while CD19 probe identified the same 3.1 kb mRNA in the CD19-ETA lane and a 2.0 kb mRNA in the CD19 lane. *A psbA* cDNA probe recognized the 1.4 kb *psbA* mRNA in both the wt and CD 19 strains, but not in the ETA or CD19-ETA lanes, confirming that both ETA and CD19-ETA integration resulted in complete deletion of the endogenous *psbA* gene (Manuell et al., 2007).

### EXAMPLE V

### ANALYSIS OF CD19, ETA, AND CD19-ETA PROTEIN ACCUMULATION IN TRANSGENIC C. REINHARDTII CHLOROPLASTS

Protein accumulation in transgenic lines was monitored by Western blot analysis. Twenty µg of total soluble protein (tsp) from wt and the transgenic lines was separated by SDS-PAGE and blotted to nitrocellulose membrane. Blots were hybridized with either an anti-ETA antibody (Figure 6, left panel) or anti-flag antibody (Figure 6, right panel). The anti-ETA antisera recognized a protein of 42 kDa in the ETA transgenic line and a protein of 71 kDa in the CD 19-ETA in the transgenic line. The anti-Flag antisera recognized the same two proteins, as well as the 30 kDa CD19 protein. Additional bands (likely degradation products) were detectable with anti-Flag in the CD19-ETA and ETA lanes.

### EXAMPLE VI

### BIOACTIVITY OF C. REINHARDTII CHLOROPLAST EXPRESSED EXOTOXIN A PROTEIN IN VITRO

*In vitro* ADP-ribosyltransferase assays were performed to detect exotoxin A-specific ribosylation of elongation factor 2 (eEF2) using purified eEF2 from wheat germ and radio-labeled NAD⁺. As shown in Figure 7, the 93 kDa eEF2 is labeled with ADP from NAD⁺ when treated with purified ETA protein expressed in *E. coli* (lane 1), and when treated with chloroplast expressed and purified ETA protein expressed and purified ETA (lane 3) or CD19-ETA (lane 4). No labeled eEF2 was observed in controls lacking exotoxin A.

### EXAMPLE VII

### CD19 BINDING AND CELL KILLING ABILITY OF THE CD19-ETA FUSION PROTEIN

CD19-ETA binding to CD19-positive human cells was measured using flow cytometry and a fluorescently labeled secondary antibody directed against the Flag epitope found on the carboxy end of the CD19-ETA fusion protein. The human immortalized Ramos B-cell line, and activated human peripheral blood lymphocytes (PBLs) both express CD19. Increasing concentrations of CD19-ETA were added to both Ramos and PBL cells followed by the addition of FITC labeled anti-Flag antibodies, after which the cells were analyzed by flow cytometry. As shown in Figure 8, a concentration-dependent shift in fluorescence was observed in both cell types, demonstrating that B-cells were bound by the CD19-ETA in proportion to the amount of fusion protein added.

To determine if the CD19-ETA bound to the cells was endocytosed and killed the cells, apoptosis was measured using annexin A5 staining. Annexin A5 detects phosphatidylserine on the cell surface, a marker associated with programmed cell death (Koopman et al., 1994). Conjugation of annexin A5 with FITC thus reveals cell killing by increased fluorescence of cells expressing the annexin A5 ligand. As shown in Figure 9, treatment of PBLs with the CD19 scFv alone had no effect on fluorescence even after a 24 hour incubation. Treatment with exotoxin A domain alone also failed to induce cell killing. However, treatment of PBLs with increasing amounts of CD 19-ETA resulted in increased fluorescence, indicating that the CD19-ETA, but not CD 19 or ETA alone, induces phosphatidylserine suggestive of concentration-dependent cell killing.

### EXAMPLE VIII

### PRODUCTION OF A RIBOSOME INACTIVATING PROTEIN, GELONIN, IN ALGAL CHLOROPLASTS

To determine if eukaryotic toxins in addition to ETA could also be produced in algal chloroplasts a gene encoding a codon optimized ribosome inactivating protein, gelonin was generated. Not to be bound by theory, however, gelonin seems to inactivate 80S eukaryotic ribosomes resulting in cell death, but does not inactivate bacterial ribosomes or chloroplast ribosomes. An SAA-gelonin fusion protein was constructed for expression in algal chloroplasts. Figure 10 shows the nucleotide and amino acids sequence of the SAA-nGelonin fusion protein (SEQ ID NOS:6 and 7). Amino acid residues 1 to 113 define the codon optimized bovine serum amyloid A 3 protein, amino acid residues 114 to 119 define the flexible peptide linker, amino acid residues 120 to 128 define a TEV protease site, amino acid residues 129 to 379 define native Gelonin, and amino acid residues 380 to 405 at the carboxy terminus define the FLAG epitope tag. Figure 11 shows a western blot analysis of recombinant rGelonin and SAA-nGelonin protein accumulation in *C*. *reinhardtii* transgenic chloroplasts. Total proteins from wt, a transgenic line expressing rGel, and a dilution series of proteins from a transgenic line expressing SAA-nGelonin are shown. The proteins were blotted to membranes and decorated with anti-FLAG (right panel) antisera. *In vitro* activity assay of isolated chloroplast expressed SAA-nGelonin is shown in figure 12. Lane 2 shows a control primer extension product. Lane 3 shows primer extension with no added protein, lane 4 shows primer extension with bacterially expressed rGelonin added, and lane 6 shows primer extension with purified SAA-nGelonin added. These data demonstrate that eukaryotic 80S ribosome inactivating proteins can be expressed in algal chloroplasts, and that chloroplast are capable of expressing a variety of eukaryotic toxins.

### EXAMPLE IX

### Fc-ETA FUSION PROTEIN

The amino acid sequence for ETA to be used will be as above. Briefly, a chloroplast biased nucleotide sequence is generated which encodes ETA (see Franklin et al. Plant J (2002) 30:733-744, Mayfield et al., Proc Natl Acad Sci USA (2003) 100:438-442, Mayfield et al., Plant J (2004) 37:449-458) using PCR based oligonucleotide gene assembly (Stemmer et al., Gene (1995) 164:49-53). Once assembled, the sequence will be linked the hinge, C_{H}2-C_{H}3 domains of heavy chain hIgG1 using an in frame amino acid linker (low complexity) located between the carboxy terminus of the ETA and amino terminus of the Fc region. The fusion protein may be purified by Protein A or Protein G affinity chromatography.

### EXAMPLE X

### Fc-GELONIN FUSION PROTEIN

The amino acid sequence for gelonin to be used will be as above. Briefly, a chloroplast biased nucleotide sequence is generated which encodes gelonin (see Franklin et al. Plant J (2002) 30:733-744, Mayfield et al., Proc Natl Acad Sci USA (2003) 100:438-442, Mayfield et al., Plant J (2004) 37:449-458) using PCR based oligonucleotide gene assembly (Stemmer et al., Gene (1995) 164:49-53). Once assembled, the sequence will be linked the hinge, C_{H}2-C_{H}3 domains of heavy chain hIgG1 using an in frame amino acid linker (low complexity) located between the carboxy terminus of the gelonin and amino terminus of the Fc region. Again, the fusion protein may be purified by Protein A or Protein G affinity chromatography.

### EXAMPLE XI

### IN VIVO CD19-ETA IMMUNOTOXIN FUSION ACTIVITY

The bioactivity of CD19-ETA and other immunotoxin fusions with respect to clearance and cell killing is analyzed in an implanted human B cell lymphoma animal model. The Ramos cell line is a well-established model for human B cell lymphomas and has proven useful to provide a clear proof of concept that the algae-produced CD19-ETA toxin construct binds and efficiently kills the Ramos cells *in vitro.* Cell death occurs within 24 hours of exposure to the fusion protein. To establish the proof of killing activity *in vivo* a Ramos cell line engineered to constitutively express the firefly luciferase gene will be created. These luciferase-labeled Ramos cells (Ramos/luc) will be implanted in a single Matrigel^{™} scaffold in the abdominal wall of immunodeficient NOD/SCID mice to form a discrete tumor. Intraperitoneal injection of the luciferase substrate, luciferin, will result in a light emission from the labeled tumor cells that is imaged using a Xenogen instrument. The advantages of this approach is that imaging is done on anesthetized, live animals allowing the tumor's progression or destruction to be followed serially over time and as a function of CD 19-ETA dose with a high degree of accuracy and sensitivity. With this technology, multiple animals including controls can be readily imaged in a single experiment. A second approach will be to implant the Ramos/luc by injection directly into the blood stream via tail vein injection. This results in a general dissemination of the lymphoma cells, particularly to spleen, lungs and liver, very much like a human clinical presentation of Stage III or IV lymphoma. The Xenogen luciferase imaging technology is also well-suited to detection and measurement of this type of multiple small tumor metastases. The objectives of these studies will be to demonstrate the capability of the CD19-ETA construct to kill both a discrete tumor and disseminated disease, and to establish the total required dose, time frame and correlations with achieved serum levels of the fusion protein to achieve these effects. Another critical question for these preclinical studies is the ability of the CD19-ETA construct to efficiently enter and kill lymphoma cells within discrete tissue compartments such as spleen and liver. As additional constructs are considered, the issue of how additional toxin candidates and increasingly larger and more complex proteins function in tissue compartments becomes critical, because increased *in vitro* binding or killing efficiency is not useful if the new constructs cannot readily penetrate to the local site of the tumor cell *in vivo.* Additional studies include injection of another B cell lymphoma line and a survey of implanting multiple naturally occurring B cell lymphoma cells derived from human patients. Studies will also involve testing the immune response to this therapeutic protein. The initial studies will be done in immunodeficient NOD/SCID mice that mount no immune response to either the implantation of the human tumor cells or the CD19-ETA. Thus, studies of immune responses to the fusion protein will be done in fully immunocompetent mouse strains such as C57/B16, C3Hej and Balb/c. It is expected that following administration of a therapeutically effective dose of anti-CD19-ETA immunotoxin a concentration dependent killing of human lymphoma cells and concomitant loss of luciferase luminescence is observed.

### EXAMPLE XII

### FULL-LENGTH ANTIBODY - TOXIN FUSIONS

Chloroplasts are eukaryotic organelles that contain a number of chaperones normally used for folding and assembly of complex photosynthetic proteins imported into chloroplast from the cytoplasm. Chloroplasts have also been shown to have protein disulfide isomerases, and plastids have been shown to be able to form correct disulfide bonds in recombinant human somatotropin, and to assemble correctly disulfide linked complex human antibodies, processes that bacterial are generally unable to complete. The ability to assemble complex human antibodies in an environment that allows for toxin synthesis and accumulation, should allow for the synthesis and assembly of full-length human antibody-toxin fusion proteins. Full length heavy chain protein genes, from antibodies directed against CD 19, CD22, or any appropriate cell surface antigen, will be constructed with a restriction site on the carboxy end of the heavy chain coding region to allow for the inframe fusion of a toxin domain. The resulting heavy chain-toxin protein gene will be transformed in plastids, along with a corresponding light chain gene, so that both proteins will be synthesized within the same plastid. Simultaneous expression of light chain and heavy chain-toxin proteins in chloroplasts will allow for the assembly of a full length antibody containing a toxin domain on the carboxy end of the heavy chain protein. Expression in this way should allow for unobstructed binding to the appropriate antigen from the variable regions of the light and heavy chain proteins as well as increased stability of the antibody-toxin protein brought about by the stabilizing effects of the heavy chain constant domains. Similar constructs will be made using a Fab fragment of the heavy chain with an appropriate site on the carboxy end of the heavy chain protein to fuse an inframe toxin domain. Co-expression of a Fab heavy chain-toxin protein with the appropriate light chain protein should result in a Fab-toxin fusion protein containing two antigen binding domains and two toxin domains, resulting in a potentially superior cell binding and killing molecule.

### EXAMPLE XIII

### CD19 scFv-GELONIN - TOXIN FUSIONS

A CD19 scFv-Gelonin fusion protein was generated as shown in Figure 14 as described herein (SEQ ID NOS:10 and 11, respectively). Amino acid residues 1 to 115 define the variable regions of the light chain, amino acid residues 116 to 135 define the flexible peptide linker, amino acid residues 136 to 264 define the variable region of the heavy chain, amino acid residues 265 to 276 define the flexible peptide linker, amino acid residues 277 to 527 define native Gelonin, and amino acid residues 528 to 556 at the carboxy terminus define the FLAG epitope tag.

An *in vitro* gelonin assay was performed using the algal expressed CD 19 scFv-Gelonin fusion protein. Gelonin activity is assayed by primer extension with radio-labeled primer. Yeast ribosomes were treated with purified recombinant gelonin, CD19:Gelonin, or untreated (no protein). Active gelonin will cleave the rRNA within the ricin loop. After treatment rRNA is isolated and used as a template for primer extension. 'Experimental' primers will give a product if gelonin activity is present (Figure 15A). 'Control' primers will give a product (Figure 15B) if rRNA is present.

As shown in Figure 16, the algal expressed CD19 scFv-Gelonin fusion protein was purified. Figure 16A shows a Western blot of starting material, purified by FLAG affinity from crude algae lysate, before and after concentration (S1 and S2 respectively), then elutions from desalting column. Figure 16B shows the elution profile from desalting column. Darker line shows UV absorbance, lighter line shows conductivity (salt). Figure 16C shows a Western blot of purified desalted samples. Elutions 2-10 from desalting column were pooled (lane 1) and concentrated (lane 2), and filtered (lane 4).

### EXAMPLE XIV

### CD19 scFv- CH2-ETA - TOXIN FUSIONS

A CD19 scFv-CH2-ETA fusion protein was generated as shown in Figure 17 as described herein (SEQ ID NOS:12 and 13, respectively). Amino acid residues 1 to 261 define the variable regions of the light chain, amino acid residues 262 to 381 define the CH2 constant domain, amino acid residues 382 to 772 define Exotoxin A, amino acid residues 773 to 780 define a TEV cleavage site, amino acid residues 781 to 786 define the flexible peptide linker, and amino acid residues 782 to 791 at the carboxy terminus define the FLAG epitope tag.

Figure 18 shows algal expression of an anti-CD19-scFv-heavy chain CH2 domain-exotoxin A chimeric protein. Four transgenic lines, 32-1, 34-3, 41-4 and 45-1 were analyzed by western blot analysis for the accumulation of the chimeric protein. Protein from non-transformed wild type cells (Wt) was loaded in Lane 1. The chimeric antibody-toxin protein (arrowhead) accumulates as a soluble protein at the correct molecular weight (85 kD) in at least three of the transgenic lines, 32-1, 41-4 and 45-1. The chimeric protein was visualized using an anti-ETA antibody.

## Claims

1. A nucleic acid construct comprising in operable linkage:
a) nucleic acid signaling elements for homologous recombination and expression of a fusion protein in a chloroplast of a plant or eukaryotic alga; and
b) a first polynucleotide sequence encoding a first polypeptide and a second polynucleotide sequence encoding a toxin functional in a eukaryotic cell, wherein the first polypeptide encodes a binding domain specific for a cluster of differentiation "CD" cell surface marker, and wherein the first and second polynucleotide sequences are expressed as a fusion protein in the chloroplast of the plant or eukaryotic alga, wherein the toxin is a bacterial or plant derived toxin.

2. The construct of claim 1, wherein the binding domain comprises an antibody or an antigen binding fragment thereof.

3. The construct of claim 2, wherein the antibody or the antigen binding fragment thereof is selected from a complete antibody, and an Fc region.

4. The construct of claim 1, wherein the toxin is an endotoxin or an exotoxin.

5. The construct of claim 1, wherein the toxin is exotoxin A or gelonin.

6. The nucleic acid construct of claim 1, wherein the cell surface marker is selected from the group consisting of, CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD 18, CD 19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d, and CD66e.

7. A plant or algal chloroplast comprising the construct of any one of the preceding claims.

8. A plant or algal cell comprising the chloroplast of claim 7.

9. A method for producing a functional fusion protein comprising,
a) contacting a chloroplast of a plant or eukaryotic alga with at least one expression construct, the expression construct comprising in operable linkage, (i) nucleic acid signal elements for homologous recombination and expression of the fusion protein in the chloroplast of the plant or eukaryotic alga, and (ii) a first polynucleotide sequence encoding a first polypeptide comprising a binding domain specific for a cluster of differentiation "CD" cell surface marker, and a second polynucleotide sequence encoding a toxin functional in a eukaryotic cell, the toxin being a bacterial or plant derived toxin;
b) allowing the construct to integrate into the chloroplast genome; and
c) expressing the first and second polynucleotide sequences as a fusion protein in the chloroplast of the plant or eukaryotic alga.

10. The method of claim 9, wherein the binding domain comprises an antibody or an antigen binding fragment thereof.

11. The method of claim 10, wherein the antibody or the antigen binding fragment thereof is selected from a complete antibody, and an Fc region.

12. The method of claim 9, wherein the toxin is an endotoxin or an exotoxin.

13. The method of claim 9, wherein the toxin is exotoxin A or gelonin.

14. The method of claim 9, wherein the cell surface marker is selected from the group consisting of CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49c, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d, and CD66e.

15. The method of claim 9, further comprising:
d) isolating the fusion protein from the plant or eukaryotic alga.

## Patentansprüche

1. Nukleinsäurekonstrukt, umfassend in operativer Verknüpfung:
a) Nukleinsäuresignalisierungselemente für homologe Rekombination und Expression eines Fusionsproteins in einem Chloroplasten einer Pflanze oder eukaryontischen Alge; und
b) eine erste Polynukleotidsequenz, die ein erstes Polypeptid kodiert, und eine zweite Polynukleotidsequenz, die ein Toxin kodiert, das in einer eukaryontischen Zelle funktional ist, wobei das erste Polypeptid eine Bindungsdomäne kodiert, die für einen Differenzierungscluster("Cluster of Differentiation", CD)-Zelloberflächenmarker spezifisch ist, und wobei die erste und die zweite Polynukleotidsequenz als Fusionsprotein in dem Chloroplasten der Pflanze oder der eukaryontischen Alge exprimiert sind, wobei es sich bei dem Toxin um ein bakterielles oder von Pflanzen stammendes Toxin handelt.

2. Konstrukt nach Anspruch 1, wobei die Bindungsdomäne einen Antikörper oder ein Antigenbindungsfragment davon umfasst.

3. Konstrukt nach Anspruch 2, wobei der Antikörper oder das Antigenbindungsfragment davon aus einem vollständigen Antikörper und einer Fc-Region ausgewählt ist.

4. Konstrukt nach Anspruch 1, wobei es sich bei dem Toxin um ein Endotoxin oder ein Exotoxin handelt.

5. Konstrukt nach Anspruch 1, wobei es sich bei dem Toxin um Exotoxin A oder Gelonin handelt.

6. Nukleinsäurekonstrukt nach Anspruch 1, wobei der Zelloberflächenmarker aus der Gruppe ausgewählt ist, die aus CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d und CD66e besteht.

7. Pflanzen- oder Algenchloroplast, umfassend das Konstrukt nach einem der vorhergehenden Ansprüche.

8. Pflanzen- oder Algenzelle, umfassend den Chloroplasten nach Anspruch 7.

9. Verfahren zum Herstellen eines funktionalen Fusionsproteins, das Folgende umfasst:
a) In-Berührung-bringen eines Chloroplasten einer Pflanze oder eukaryontischen Alge mit mindestens einem Expressionskonstrukt, wobei das Expressionskonstrukt (i) Nukleinsäuresignalisierungselemente für homologe Rekombination und Expression des Fusionsproteins im Chloroplasten der Pflanze oder eukaryontischen Alge; und (ii) eine erste Polynukleotidsequenz, die ein erstes Polypeptid, umfassend eine Bindungsdomäne, die für einen Differenzierungscluster("Cluster of Differentiation", CD)-Zelloberflächenmarker spezifisch ist, kodiert, und eine zweite Polynukleotidsequenz, die ein Toxin kodiert, das in einer eukaryontischen Zelle funktional ist, wobei es sich bei dem Toxin um ein bakterielles oder von Pflanzen stammendes Toxin handelt, in operativer Verknüpfung umfasst;
b) es dem Konstrukt ermöglichen, sich in das Chloroplastengenom zu integrieren; und
c) Exprimieren der ersten und der zweiten Polynukleotidsequenz als Fusionsprotein in dem Chloroplasten der Pflanzen oder eukaryontischen Alge.

10. Verfahren nach Anspruch 9, wobei die Bindungsdomäne einen Antikörper oder ein Antigenbindungsfragment davon umfasst.

11. Verfahren nach Anspruch 10, wobei der Antikörper oder das Antigenbindungsfragment davon aus einem vollständigen Antikörper und einer Fc-Region ausgewählt ist.

12. Verfahren nach Anspruch 9, wobei es sich bei dem Toxin um ein Endotoxin oder ein Exotoxin handelt.

13. Verfahren nach Anspruch 9, wobei es sich bei dem Toxin um Exotoxin A oder Gelonin handelt.

14. Verfahren nach Anspruch 9, wobei der Zelloberflächenmarker aus der Gruppe ausgewählt ist, die aus CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD66a, CD66b, CD66c, CD66d und CD66e besteht.

15. Verfahren nach Anspruch 9, ferner umfassend:
d) Isolieren des Fusionsproteins aus der Pflanze oder eukaryontischen Alge.

## Revendications

1. Construction d'acide nucléique comprenant en liaison fonctionnelle :
a) des éléments de signalisation d'un acide nucléique pour une recombinaison homologue et l'expression d'une protéine de fusion dans un chloroplaste d'une plante ou d'une algue eucaryote ; et
b) une première séquence polynucléotidique codant pour un premier polypeptide et une deuxième séquence polynucléotidique codant pour une toxine fonctionnelle dans une cellule eucaryote, dans laquelle le premier polypeptide code pour un domaine de liaison spécifique d'un cluster de différenciation marqueur de surface cellulaire « CD », et dans laquelle les première et deuxième séquences polynucléotidiques sont exprimées en tant que protéine de fusion dans le chloroplaste de la plante ou de l'algue eucaryote, dans laquelle la toxine est une toxine bactérienne ou dérivée d'une plante.

2. Construction selon la revendication 1, dans laquelle le domaine de liaison comprend un anticorps ou un fragment se liant à un antigène à celui-ci.

3. Construction selon la revendication 2, dans laquelle l'anticorps ou le fragment se liant à un antigène de celui-ci est choisi parmi un anticorps complet et une région Fc.

4. Construction selon la revendication 1, dans laquelle la toxine est une endotoxine ou une exotoxine.

5. Construction selon la revendication 1, dans laquelle la toxine est une exotoxine A ou une gélonine.

6. Construction d'acide nucléique selon la revendication 1, dans laquelle le marqueur de surface cellulaire est choisi dans le groupe comprenant CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, 62P, CD63, CD64, CD65, CD66a, CD66b, CD66c, CD66d et CD66e.

7. Chloroplaste de plante ou d'algue comprenant la construction selon l'une quelconque des revendications précédentes.

8. Cellule de plante ou d'algue comprenant le chloroplaste selon la revendication 7.

9. Procédé de production d'une protéine de fusion fonctionnelle comprenant
a) la mise en contact d'un chloroplaste d'une plante ou d'une algue eucaryote avec au moins une construction d'expression, la construction d'expression comprenant, en liaison fonctionnelle, (i) des éléments de signal d'un acide nucléique pour la recombinaison homologue et l'expression de la protéine de fusion dans le chloroplaste de la plante ou de l'algue eucaryote, et (ii) une première séquence polynucléotidique codant pour un premier polypeptide comprenant un domaine de liaison spécifique d'un cluster de différenciation marqueur de surface cellulaire « CD » et une deuxième séquence polynucléotidique codant pour une toxine fonctionnelle dans une cellule eucaryote, la toxine étant une toxine bactérienne ou dérivée d'une plante ;
b) la possibilité pour la construction, d'être intégrée dans le génome du chloroplaste ; et
c) l'expression des première et deuxième séquences polynucléotidiques en tant que protéine de fusion dans le chloroplaste de la plante ou d'une algue eucaryote.

10. Procédé selon la revendication 9, dans lequel le domaine de liaison comprend un anticorps ou un fragment se liant à un antigène de celui-ci.

11. Procédé selon la revendication 10, dans lequel l'anticorps ou le fragment se liant à un antigène de celui-ci est choisi parmi un anticorps complet et une région Fc.

12. Procédé selon la revendication 9, dans lequel la toxine est une endotoxine ou une exotoxine.

13. Procédé selon la revendication 9, dans lequel la toxine est l'exotoxine A ou la gélonine.

14. Procédé selon la revendication 9, dans lequel le marqueur de surface cellulaire est choisi dans le groupe comprenant CD1, CD2, CD3, CD4, CD5, CD6, CD7, CD8, CD9, CD10, CD11a, CD11b, CD11c, CD11d, CDw12 , CD13, CD14, CD15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, 62P, CD63, CD64, CD65, CD66a, CD66b, CD66c, CD66d et CD66e.

15. Procédé selon la revendication 9, comprenant en outre :
d) l'isolement de la protéine de fusion de la plante ou de l'algue eucaryote.


### SEQUENCE LISTING

<110> THE SCRIPPS RESEARCH INSTITUTE MAYFIELD, Stephen P.
<120> PRODUCTION OF CYTOTOXIC ANTIBODY-TOXIN FUSION IN EUKARYOTIC ALGAE
<130> SCRIP1880-1WO
<150> US 60/987,726
   <151> 2007-11-13
<160> 14
<170> PatentIn version 3.5
<210> 1
   <211> 289
   <212> PRT
   <213> Artificial Sequence
<220>
   <223> Synthetic construct
<400> 1
   Met Ser Ile Val Met Thr Gln Ala Ala Pro Ser Ile Pro Val Thr Pro
   1               5                   10                  15
   Gly Glu Ser Val Ser Ile Ser Cys Arg Ser Ser Lys Ser Leu Leu Asn
               20                  25                  30
   Ser Asn Gly Asn Thr Tyr Leu Tyr Trp Phe Leu Gln Arg Pro Gly Gln
           35                  40                  45
   Ser Pro Gln Leu Leu Ile Tyr Arg Met Ser Asn Leu Ala Ser Gly Val
       50                  55                  60
   Pro Asp Arg Phe Ser Gly Ser Gly Ser Gly Thr Ala Phe Thr Leu Arg
   65                  70                  75                  80
   Ile Ser Arg Val Glu Ala Glu Asp Val Gly Val Tyr Tyr Cys Met Gln
                   85                  90                  95
   His Leu Glu Tyr Pro Leu Thr Phe Gly Cys Gly Thr Lys Leu Glu Ile
               100                 105                 110
   Lys Arg Gly Gly Gly Gly Ser Gly Gly Gly Gly Ser Gly Gly Gly Gly
           115                 120                 125
   Ser Gly Gly Gly Gly Ser Gln Val Gln Leu Gln Gln Ser Gly Pro Glu
       130                 135                 140
   Leu Ile Lys Pro Gly Ala Ser Val Lys Met Ser Cys Lys Ala Ser Gly
   145                 150                 155                 160
   Tyr Thr Phe Thr Ser Tyr Val Met His Trp Val Lys Gln Lys Pro Gly
                   165                 170                 175
   Gln Cys Leu Glu Trp Ile Gly Tyr Ile Asn Pro Tyr Asn Asp Gly Thr
               180                 185                 190
   Lys Tyr Asn Glu Lys Phe Lys Gly Lys Ala Thr Leu Thr Ser Asp Lys
           195                 200                 205
   Ser Ser Ser Thr Ala Tyr Met Glu Leu Ser Ser Leu Thr Ser Glu Asp
       210                 215                 220
   Ser Ala Val Tyr Tyr Cys Ala Arg Gly Thr Tyr Tyr Tyr Gly Ser Arg
   225                 230                 235                 240
   Val Phe Asp Tyr Trp Gly Gln Gly Thr Thr Leu Thr Val Thr Val Ser
                   245                 250                 255
   Ser Ala Ser Gly Ala Gly Thr Gly Thr Cys Tyr Asp Tyr Lys Asp His
               260                 265                 270
   Asp Gly Lys Asp His Asp Ile Asp Tyr Lys Asp Asp Asp Asp Lys Ser
           275                 280                 285
   Arg
<210> 2
   <211> 1182
   <212> DNA
   <213> Pseudomonas aeruginosa
<220>
   <221> CDS
   <222> (4)..(1173)
<220>
   <221> CDS
   <222> (1177)..(1182)
<400> 2
   cat atg gca gaa ggt ggt agc cta gca gct cta act gct cac caa gct      48
       Met Ala Glu Gly Gly Ser Leu Ala Ala Leu Thr Ala His Gln Ala
       1               5                   10                  15
   tgt cac cta ccg cta gaa act ttc act cgt cat cgc caa ccg cgc ggt      96
   Cys His Leu Pro Leu Glu Thr Phe Thr Arg His Arg Gln Pro Arg Gly
                   20                  25                  30
   tgg gaa caa cta gaa caa tgt ggt tat ccg gta caa cgt cta gtt gca     144
   Trp Glu Gln Leu Glu Gln Cys Gly Tyr Pro Val Gln Arg Leu Val Ala
               35                  40                  45
   ctt tac cta gct gct cgt cta tct tgg aac caa gtt gac caa gta atc     192
   Leu Tyr Leu Ala Ala Arg Leu Ser Trp Asn Gln Val Asp Gln Val Ile
           50                  55                  60
   cgc aac gca cta gca agc cct ggt agc ggt ggt gac cta ggt gaa gct     240
   Arg Asn Ala Leu Ala Ser Pro Gly Ser Gly Gly Asp Leu Gly Glu Ala
       65                  70                  75
   atc cgc gaa caa ccg gaa caa gca cgt cta gca cta act cta gca gca      288
   Ile Arg Glu Gln Pro Glu Gln Ala Arg Leu Ala Leu Thr Leu Ala Ala
   80                  85                  90                  95
   gca gaa agc gaa cgc ttc gtt cgt caa ggt act ggt aac gac gaa gca      336
   Ala Glu Ser Glu Arg Phe Val Arg Gln Gly Thr Gly Asn Asp Glu Ala
                   100                 105                 110
   ggt gct gca aac gca gac gta gta agc cta act tgt ccg gtt gca gca      384
   Gly Ala Ala Asn Ala Asp Val Val Ser Leu Thr Cys Pro Val Ala Ala
               115                 120                 125
   ggt gaa tgt gct ggt ccg gct gac agc ggt gac gca cta cta gaa cgc      432
   Gly Glu Cys Ala Gly Pro Ala Asp Ser Gly Asp Ala Leu Leu Glu Arg
           130                 135                 140
   aac tat cct act ggt gct gaa ttc ctt ggt gac ggt ggt gac gtt agc      480
   Asn Tyr Pro Thr Gly Ala Glu Phe Leu Gly Asp Gly Gly Asp Val Ser
       145                 150                 155
   ttc agc act cgc ggt acg caa aac tgg acc gtg gaa cgc cta ctt caa      528
   Phe Ser Thr Arg Gly Thr Gln Asn Trp Thr Val Glu Arg Leu Leu Gln
   160                 165                 170                 175
   gct cac cgc caa cta gaa gaa cgc ggt tat gta ttc gtt ggt tac cac      576
   Ala His Arg Gln Leu Glu Glu Arg Gly Tyr Val Phe Val Gly Tyr His
                   180                 185                 190
   ggt act ttc ctt gaa gct gct caa agc atc gtt ttc ggt ggt gta cgc      624
   Gly Thr Phe Leu Glu Ala Ala Gln Ser Ile Val Phe Gly Gly Val Arg
               195                 200                 205
   gct cgc agc caa gac ctt gac gct atc tgg cgc ggt ttc tat atc gca      672
   Ala Arg Ser Gln Asp Leu Asp Ala Ile Trp Arg Gly Phe Tyr Ile Ala
           210                 215                 220
   ggt gat ccg gct cta gca tac ggt tac gca caa gac caa gaa cct gac      720
   Gly Asp Pro Ala Leu Ala Tyr Gly Tyr Ala Gln Asp Gln Glu Pro Asp
       225                 230                 235
   gca cgc ggt cgt atc cgc aac ggt gca cta cta cgt gtt tat gta ccg      768
   Ala Arg Gly Arg Ile Arg Asn Gly Ala Leu Leu Arg Val Tyr Val Pro
   240                 245                 250                 255
   cgc tct agc cta ccg ggt ttc tac cgc act agc cta act cta gca gct      816
   Arg Ser Ser Leu Pro Gly Phe Tyr Arg Thr Ser Leu Thr Leu Ala Ala
                   260                 265                 270
   ccg gaa gct gct ggt gaa gtt gaa cgt cta atc ggt cat ccg cta ccg      864
   Pro Glu Ala Ala Gly Glu Val Glu Arg Leu Ile Gly His Pro Leu Pro
               275                 280                 285
   cta cgc cta gac gca atc act ggt cct gaa gaa gaa ggt ggt cgc cta      912
   Leu Arg Leu Asp Ala Ile Thr Gly Pro Glu Glu Glu Gly Gly Arg Leu
           290                 295                 300
   gaa act att ctt ggt tgg ccg cta gca gaa cgc act gta gta att cct      960
   Glu Thr Ile Leu Gly Trp Pro Leu Ala Glu Arg Thr Val Val Ile Pro
       305                 310                 315
   tct gct atc cct act gac ccg cgc aac gtt ggt ggt gac ctt gac ccg     1008
   Ser Ala Ile Pro Thr Asp Pro Arg Asn Val Gly Gly Asp Leu Asp Pro
   320                 325                 330                 335
   tca agc atc cct gac aag gaa caa gct atc agc gca cta ccg gac tac     1056
   Ser Ser Ile Pro Asp Lys Glu Gln Ala Ile Ser Ala Leu Pro Asp Tyr
                   340                 345                 350
   gca agc caa cct ggt aaa ccg ccg cgc gaa gac cta aag ggt acc tgt        1104
   Ala Ser Gln Pro Gly Lys Pro Pro Arg Glu Asp Leu Lys Gly Thr Cys
               355                 360                 365
   tac gat tat aaa gat cac gat ggt gat tac aaa gat cac gat att gat        1152
   Tyr Asp Tyr Lys Asp His Asp Gly Asp Tyr Lys Asp His Asp Ile Asp
           370                 375                 380
   tat aaa gat gat gat gat aaa taa tct aga                                1182
   Tyr Lys Asp Asp Asp Asp Lys     Ser Arg
       385                 390
<210> 3
   <211> 392
   <212> PRT
   <213> Pseudomonas aeruginosa
<400> 3
   Met Ala Glu Gly Gly Ser Leu Ala Ala Leu Thr Ala His Gln Ala Cys
   1               5                   10                  15
   His Leu Pro Leu Glu Thr Phe Thr Arg His Arg Gln Pro Arg Gly Trp
               20                  25                  30
   Glu Gln Leu Glu Gln Cys Gly Tyr Pro Val Gln Arg Leu Val Ala Leu
           35                  40                  45
   Tyr Leu Ala Ala Arg Leu Ser Trp Asn Gln Val Asp Gln Val Ile Arg
       50                  55                  60
   Asn Ala Leu Ala Ser Pro Gly Ser Gly Gly Asp Leu Gly Glu Ala Ile
   65                  70                  75                  80
   Arg Glu Gln Pro Glu Gln Ala Arg Leu Ala Leu Thr Leu Ala Ala Ala
                   85                  90                  95
   Glu Ser Glu Arg Phe Val Arg Gln Gly Thr Gly Asn Asp Glu Ala Gly
               100                 105                 110
   Ala Ala Asn Ala Asp Val Val Ser Leu Thr Cys Pro Val Ala Ala Gly
           115                 120                 125
   Glu Cys Ala Gly Pro Ala Asp Ser Gly Asp Ala Leu Leu Glu Arg Asn
       130                 135                 140
   Tyr Pro Thr Gly Ala Glu Phe Leu Gly Asp Gly Gly Asp Val Ser Phe
   145                 150                 155                 160
   Ser Thr Arg Gly Thr Gln Asn Trp Thr Val Glu Arg Leu Leu Gln Ala
                   165                 170                 175
   His Arg Gln Leu Glu Glu Arg Gly Tyr Val Phe Val Gly Tyr His Gly
               180                 185                 190
   Thr Phe Leu Glu Ala Ala Gln Ser Ile Val Phe Gly Gly Val Arg Ala
           195                 200                 205
   Arg Ser Gln Asp Leu Asp Ala Ile Trp Arg Gly Phe Tyr Ile Ala Gly
       210                 215                 220
   Asp Pro Ala Leu Ala Tyr Gly Tyr Ala Gln Asp Gln Glu Pro Asp Ala
   225                 230                 235                 240
   Arg Gly Arg Ile Arg Asn Gly Ala Leu Leu Arg Val Tyr Val Pro Arg
                   245                 250                 255
   Ser Ser Leu Pro Gly Phe Tyr Arg Thr Ser Leu Thr Leu Ala Ala Pro
               260                 265                 270
   Glu Ala Ala Gly Glu Val Glu Arg Leu Ile Gly His Pro Leu Pro Leu
           275                 280                 285
   Arg Leu Asp Ala Ile Thr Gly Pro Glu Glu Glu Gly Gly Arg Leu Glu
       290                 295                 300
   Thr Ile Leu Gly Trp Pro Leu Ala Glu Arg Thr Val Val Ile Pro Ser
   305                 310                 315                 320
   Ala Ile Pro Thr Asp Pro Arg Asn Val Gly Gly Asp Leu Asp Pro Ser
                   325                 330                 335
   Ser Ile Pro Asp Lys Glu Gln Ala Ile Ser Ala Leu Pro Asp Tyr Ala
               340                 345                 350
   Ser Gln Pro Gly Lys Pro Pro Arg Glu Asp Leu Lys Gly Thr Cys Tyr
           355                 360                 365
   Asp Tyr Lys Asp His Asp Gly Asp Tyr Lys Asp His Asp Ile Asp Tyr
       370                 375                 380
   Lys Asp Asp Asp Asp Lys Ser Arg
   385                 390
<210> 4
   <211> 2022
   <212> DNA
   <213> Artificial Sequence
<220>
   <223> Synthetic construct
<400> 4
   catatgatag ttatgacaca agctgcacca tctattcctg ttactcctgg agaatcagta        60
   tcaatctcat gtcgttctag taaaagtctt ctgaatagta atggtaacac ttacttatat       120
   tggttcctgc aacgtccagg ccaatctcct caacttctga tttatcgtat gtcaaacctt       180
   gcttcaggtg ttccagaccg tttcagtggt agtggttcag gaactgcttt cacactgaga       240
   atcagtagag tagaagctga agatgtaggt gtttattact gtatgcaaca tttagaatat       300
   cctcttactt tcggttgtgg tacaaaactg gaaatcaaac gaggaggagg aggatctgga       360
   ggaggaggat ctggaggagg cggatcagga ggaggtggtt cacaagttca acttcaacaa       420
   tctggacctg aactgattaa acctggtgct tcagtaaaaa tgtcatgtaa agcttctgga       480
   tacacattca ctagctatgt tatgcactgg gtaaaacaaa aacctggtca atgtcttgaa       540
   tggattggat atattaatcc ttacaatgat ggtactaaat acaatgaaaa attcaaaggt       600
   aaagctacac tgacttcaga caaatcatca agcacagctt acatggaact tagcagcctg       660
   acatctgaag actctgcagt ttattactgt gcaagaggta cttattacta cggtagtcgt       720
   gtatttgact actggggcca aggtacaact cttacagtta cagtttcatc tgcttctggt       780
   gctggtacca gttctggtgg cggtggcagt agtggtggtg gcggtagtag tggtggcggt       840
   ggcatggcag aaggtggtag cctagcagct ctaactgctc accaagcttg tcacctaccg       900
   ctagaaactt tcactcgtca tcgccaaccg cgcggttggg aacaactaga acaatgtggt       960
   tatccggtac aacgtctagt tgcactttac ctagctgctc gtctatcttg gaaccaagtt      1020
   gaccaagtaa tccgcaacgc actagcaagc cctggtagcg gtggtgacct aggtgaagct      1080
   atccgcgaac aaccggaaca agcacgtcta gcactaactc tagcagcagc agaaagcgaa      1140
   cgcttcgttc gtcaaggtac tggtaacgac gaagcaggtg ctgcaaacgc agacgtagta      1200
   agcctaactt gtccggttgc agcaggtgaa tgtgctggtc cggctgacag cggtgacgca      1260
   ctactagaac gcaactatcc tactggtgct gaattccttg gtgacggtgg tgacgttagc      1320
   ttcagcactc gcggtacgca aaactggacc gtggaacgcc tacttcaagc tcaccgccaa      1380
   ctagaagaac gcggttatgt attcgttggt taccacggta ctttccttga agctgctcaa      1440
   agcatcgttt tcggtggtgt acgcgctcgc agccaagacc ttgacgctat ctggcgcggt      1500
   ttctatatcg caggtgatcc ggctctagca tacggttacg cacaagacca agaacctgac      1560
   gcacgcggtc gtatccgcaa cggtgcacta ctacgtgttt atgtaccgcg ctctagccta      1620
   ccgggtttct accgcactag cctaactcta gcagctccgg aagctgctgg tgaagttgaa      1680
   cgtctaatcg gtcatccgct accgctacgc ctagacgcaa tcactggtcc tgaagaagaa      1740
   ggtggtcgcc tagaaactat tcttggttgg ccgctagcag aacgcactgt agtaattcct      1800
   tctgctatcc ctactgaccc gcgcaacgtt ggtggtgacc ttgacccgtc aagcatccct      1860
   gacaaggaac aagctatcag cgcactaccg gactacgcaa gccaacctgg taaaccgccg      1920
   cgcgaagacc taaagggtac atgttacgat tataaagatc acgatggtga ttacaaagat      1980
   cacgatattg attataaaga tgatgatgat aaataatcta ga                        2022
<210> 5
   <211> 670
   <212> PRT
   <213> Artificial Sequence
<220>
   <223> Synthetic construct
<400> 5
   Met Ile Val Met Thr Gln Ala Ala Pro Ser Ile Pro Val Thr Pro Gly
   1               5                   10                  15
   Glu Ser Val Ser Ile Ser Cys Arg Ser Ser Lys Ser Leu Leu Asn Ser
               20                  25                  30
   Asn Gly Asn Thr Tyr Leu Tyr Trp Phe Leu Gln Arg Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Ala Phe Thr Leu Arg Ile
   65                  70                  75                  80
   Ser Arg Val Glu Ala Glu Asp Val Gly Val Tyr Tyr Cys Met Gln His
                   85                  90                  95
   Leu Glu Tyr Pro Leu Thr Phe Gly Cys Gly Thr Lys Leu Glu Ile Lys
               100                 105                 110
   Arg Gly Gly Gly Gly Ser Gly Gly Gly Gly Ser Gly Gly Gly Gly Ser
           115                 120                 125
   Gly Gly Gly Gly Ser Gln Val Gln Leu Gln Gln Ser Gly Pro Glu Leu
       130                 135                 140
   Ile Lys Pro Gly Ala Ser Val Lys Met Ser Cys Lys Ala Ser Gly Tyr
   145                 150                 155                 160
   Thr Phe Thr Ser Tyr Val Met His Trp Val Lys Gln Lys Pro Gly Gln
                   165                 170                 175
   Cys Leu Glu Trp Ile Gly Tyr Ile Asn Pro Tyr Asn Asp Gly Thr Lys
               180                 185                 190
   Tyr Asn Glu Lys Phe Lys Gly Lys Ala Thr Leu Thr Ser Asp Lys Ser
           195                 200                 205
   Ser Ser Thr Ala Tyr Met Glu Leu Ser Ser Leu Thr Ser Glu Asp Ser
       210                 215                 220
   Ala Val Tyr Tyr Cys Ala Arg Gly Thr Tyr Tyr Tyr Gly Ser Arg Val
   225                 230                 235                 240
   Phe Asp Tyr Trp Gly Gln Gly Thr Thr Leu Thr Val Thr Val Ser Ser
                   245                 250                 255
   Ala Ser Gly Ala Gly Thr Ser Ser Gly Gly Gly Gly Ser Ser Gly Gly
   260 265 270
   Gly Gly Ser Ser Gly Gly Gly Gly Met Ala Glu Gly Gly Ser Leu Ala
   275 280 285
   Ala Leu Thr Ala His Gln Ala Cys His Leu Pro Leu Glu Thr Phe Thr
   290 295 300
   Arg His Arg Gln Pro Arg Gly Trp Glu Gln Leu Glu Gln Cys Gly Tyr
   305 310 315 320
   Pro Val Gln Arg Leu Val Ala Leu Tyr Leu Ala Ala Arg Leu Ser Trp
   325 330 335
   Asn Gln Val Asp Gln Val Ile Arg Asn Ala Leu Ala Ser Pro Gly Ser
   340 345 350
   Gly Gly Asp Leu Gly Glu Ala Ile Arg Glu Gln Pro Glu Gln Ala Arg
   355 360 365
   Leu Ala Leu Thr Leu Ala Ala Ala Glu Ser Glu Arg Phe Val Arg Gln
   370 375 380
   Gly Thr Gly Asn Asp Glu Ala Gly Ala Ala Asn Ala Asp Val Val Ser
   385 390 395 400
   Leu Thr Cys Pro Val Ala Ala Gly Glu Cys Ala Gly Pro Ala Asp Ser
   405 410 415
   Gly Asp Ala Leu Leu Glu Arg Asn Tyr Pro Thr Gly Ala Glu Phe Leu
   420 425 430
   Gly Asp Gly Gly Asp Val Ser Phe Ser Thr Arg Gly Thr Gln Asn Trp
   435 440 445
   Thr Val Glu Arg Leu Leu Gln Ala His Arg Gln Leu Glu Glu Arg Gly
   450 455 460
   Tyr Val Phe Val Gly Tyr His Gly Thr Phe Leu Glu Ala Ala Gln Ser
   465 470 475 480
   Ile Val Phe Gly Gly Val Arg Ala Arg Ser Gln Asp Leu Asp Ala Ile
   485 490 495
   Trp Arg Gly Phe Tyr Ile Ala Gly Asp Pro Ala Leu Ala Tyr Gly Tyr
   500 505 510
   Ala Gln Asp Gln Glu Pro Asp Ala Arg Gly Arg Ile Arg Asn Gly Ala
   515 520 525
   Leu Leu Arg Val Tyr Val Pro Arg Ser Ser Leu Pro Gly Phe Tyr Arg
       530                 535                 540
   Thr Ser Leu Thr Leu Ala Ala Pro Glu Ala Ala Gly Glu Val Glu Arg
   545                 550                 555                 560
   Leu Ile Gly His Pro Leu Pro Leu Arg Leu Asp Ala Ile Thr Gly Pro
                   565                 570                 575
   Glu Glu Glu Gly Gly Arg Leu Glu Thr Ile Leu Gly Trp Pro Leu Ala
               580                 585                 590
   Glu Arg Thr Val Val Ile Pro Ser Ala Ile Pro Thr Asp Pro Arg Asn
           595                 600                 605
   Val Gly Gly Asp Leu Asp Pro Ser Ser Ile Pro Asp Lys Glu Gln Ala
       610                 615                 620
   Ile Ser Ala Leu Pro Asp Tyr Ala Ser Gln Pro Gly Lys Pro Pro Arg
   625                 630                 635                 640
   Glu Asp Leu Lys Gly Thr Cys Tyr Asp Tyr Lys Asp His Asp Gly Asp
                   645                 650                 655
   Tyr Lys Asp His Asp Ile Asp Tyr Lys Asp Asp Asp Asp Lys
               660                 665                 670
<210> 6
   <211> 1215
   <212> DNA
   <213> Artificial Sequence
<220>
   <223> Synthetic construct
<400> 6
   catatgtggg gtacattcct taaagaagct ggtcaaggtg ctaaagacat gtggagagct 60
   taccaagaca tgaaagaagc taactaccgt ggtgcagaca aatacttcca cgctcgtggt 120
   aactatgacg ctgctcgacg tggtcctggt ggtgcttggg ctgctaaagt aatcagtaac 180
   gctagagaaa ctattcaagg tatcacagac cctcttttta aaggtatgac acgtgaccaa 240
   gtacgtgaag attctaaagc tgaccaattt gctaacgaat ggggtcgtag cggtaaagac 300
   cctaaccact tcagacctgc tggtcttcct gacaaatact caggtggtgg tggttcacca 360
   tgggaaaatt tatattttca atcaggttta gatacagttt cattttcaac aaaaggtgct 420
   acatatatta catacgttaa ctttttaaat gaattacgtg ttaaattaaa accagaaggt 480
   aattcacatg gtattccatt attacgtaaa aaatgtgatg atccaggtaa atgttttgtt 540
   ttagttgctt tatcaaatga taatggtcaa ttagctgaaa ttgctattga tgttacatca 600
   gtttatgttg ttggttatca agttcgtaat cgttcatatt tttttaaaga tgctccagat 660
   gctgcttatg aaggtttatt taaaaataca attaaaacac gtttacattt tggtggttca 720
   tatccatcat tagaaggtga aaaagcttat cgtgaaacaa cagatcttgg tattgaacca 780
   cttcgtatcg gcatcaaaaa acttgacgaa aacgcgatcg acaactacaa accaacagaa 840
   atcgcgagct ctcttcttgt tgtaatccaa atggtaagcg aagcggcacg tttcacattc 900
   atcgaaaacc aaattcgtaa caacttccaa caacgtatcc gtccagcgaa caacacaatc 960
   tctcttgaaa acaaatgggg caaacttagc ttccaaatcc gtacaagcgg tgcgaacggt 1020
   atgttcagcg aagcggtaga acttgaacgc gcgaacggca aaaaatacta cgtaactgcg 1080
   gtagatcaag taaaaccaaa aatcgcactt cttaaattcg tagacaaaga cccagaaggt 1140
   acctgttacg attataaaga tcacgatggt gattacaaag atcacgatat tgattataaa 1200
   gatgatgatg ataaa 1215
<210> 7
   <211> 405
   <212> PRT
   <213> Artificial Sequence
<220>
   <223> Synthetic construct
<400> 7
   His Met Trp Gly Thr Phe Leu Lys Glu Ala Gly Gln Gly Ala Lys Asp
   1               5                   10                  15
   Met Trp Arg Ala Tyr Gln Asp Met Lys Glu Ala Asn Tyr Arg Gly Ala
               20                  25                  30
   Asp Lys Tyr Phe His Ala Arg Gly Asn Tyr Asp Ala Ala Arg Arg Gly
           35                  40                  45
   Pro Gly Gly Ala Trp Ala Ala Lys Val Ile Ser Asn Ala Arg Glu Thr
       50                  55                  60
   Ile Gln Gly Ile Thr Asp Pro Leu Phe Lys Gly Met Thr Arg Asp Gln
   65                  70                  75                  80
   Val Arg Glu Asp Ser Lys Ala Asp Gln Phe Ala Asn Glu Trp Gly Arg
                   85                  90                  95
   Ser Gly Lys Asp Pro Asn His Phe Arg Pro Ala Gly Leu Pro Asp Lys
               100                 105                 110
   Tyr Ser Gly Gly Gly Gly Ser Pro Trp Glu Asn Leu Tyr Phe Gln Ser
           115                 120                 125
   Gly Leu Asp Thr Val Ser Phe Ser Thr Lys Gly Ala Thr Tyr Ile Thr
       130                 135                 140
   Tyr Val Asn Phe Leu Asn Glu Leu Arg Val Lys Leu Lys Pro Glu Gly
   145 150 155 160
   Asn Ser His Gly Ile Pro Leu Leu Arg Lys Lys Cys Asp Asp Pro Gly
   165 170 175
   Lys Cys Phe Val Leu Val Ala Leu Ser Asn Asp Asn Gly Gln Leu Ala
   180 185 190
   Glu Ile Ala Ile Asp Val Thr Ser Val Tyr Val Val Gly Tyr Gln Val
   195 200 205
   Arg Asn Arg Ser Tyr Phe Phe Lys Asp Ala Pro Asp Ala Ala Tyr Glu
   210 215 220
   Gly Leu Phe Lys Asn Thr Ile Lys Thr Arg Leu His Phe Gly Gly Ser
   225 230 235 240
   Tyr Pro Ser Leu Glu Gly Glu Lys Ala Tyr Arg Glu Thr Thr Asp Leu
   245 250 255
   Gly Ile Glu Pro Leu Arg Ile Gly Ile Lys Lys Leu Asp Glu Asn Ala
   260 265 270
   Ile Asp Asn Tyr Lys Pro Thr Glu Ile Ala Ser Ser Leu Leu Val Val
   275 280 285
   Ile Gln Met Val Ser Glu Ala Ala Arg Phe Thr Phe Ile Glu Asn Gln
   290 295 300
   Ile Arg Asn Asn Phe Gln Gln Arg Ile Arg Pro Ala Asn Asn Thr Ile
   305 310 315 320
   Ser Leu Glu Asn Lys Trp Gly Lys Leu Ser Phe Gln Ile Arg Thr Ser
   325 330 335
   Gly Ala Asn Gly Met Phe Ser Glu Ala Val Glu Leu Glu Arg Ala Asn
   340 345 350
   Gly Lys Lys Tyr Tyr Val Thr Ala Val Asp Gln Val Lys Pro Lys Ile
   355 360 365
   Ala Leu Leu Lys Phe Val Asp Lys Asp Pro Glu Gly Thr Cys Tyr Asp
   370 375 380
   Tyr Lys Asp His Asp Gly Asp Tyr Lys Asp His Asp Ile Asp Tyr Lys
   385 390 395 400
   Asp Asp Asp Asp Lys
   405
<210> 8
   <211> 846
   <212> DNA
   <213> Artificial Sequence
<220>
   <223> synthetic construct
<400> 8
   catatgggtt tagatacagt ttcattttca acaaaaggtg ctacatatat tacatacgtt      60
   aactttttaa atgaattacg tgttaaatta aaaccagaag gtaattcaca tggtattcca     120
   ttattacgta aaaaatgtga tgatccaggt aaatgttttg ttttagttgc tttatcaaat     180
   gataatggtc aattagctga aattgctatt gatgttacat cagtttatgt tgttggttat     240
   caagttcgta atcgttcata tttttttaaa gatgctccag atgctgctta tgaaggttta     300
   tttaaaaata caattaaaac acgtttacat tttggtggtt catatccatc attagaaggt     360
   gaaaaagctt atcgtgaaac aacagatctt ggtatcgaac cacttcgcat cggcatcaaa     420
   aaacttgacg aaaacgcgat cgacaactac aaaccaacag aaatcgcgag ctctcttctt     480
   gttgtaatcc aaatggtaag cgaagcggca cgtttcacat tcatcgaaaa ccaaattcgt     540
   aacaacttcc aacaacgtat ccgtccagcg aacaacacaa tctctcttga aaacaaatgg     600
   ggcaaactta gcttccaaat ccgtacaagc ggtgcgaacg gtatgttcag cgaagcggta     660
   gaacttgaac gcgcgaacgg caaaaaatac tacgtaactg cggtagatca agtaaaacca     720
   aaaatcgcac ttcttaaatt cgtagacaaa gacccagaag gtacctgtta cgattataaa     780
   gatcacgatg gtgattacaa agatcacgat attgattata aagatgatga tgataaataa     840
   tctaga                                                                846
<210> 9
   <211> 281
   <212> PRT
   <213> Artificial Sequence
<220>
   <223> Synthetic construct
<400> 9
   His Met Gly Leu Asp Thr Val Ser Phe Ser Thr Lys Gly Ala Thr Tyr
   1               5                   10                  15
   Ile Thr Tyr Val Asn Phe Leu Asn Glu Leu Arg Val Lys Leu Lys Pro
               20                  25                  30
   Glu Gly Asn Ser His Gly Ile Pro Leu Leu Arg Lys Lys Cys Asp Asp
           35                  40                  45
   Pro Gly Lys Cys Phe Val Leu Val Ala Leu Ser Asn Asp Asn Gly Gln
       50                  55                  60
   Leu Ala Glu Ile Ala Ile Asp Val Thr Ser Val Tyr Val Val Gly Tyr
   65                  70                  75                  80
   Gln Val Arg Asn Arg Ser Tyr Phe Phe Lys Asp Ala Pro Asp Ala Ala
   85 90 95
   Tyr Glu Gly Leu Phe Lys Asn Thr Ile Lys Thr Arg Leu His Phe Gly
   100 105 110
   Gly Ser Tyr Pro Ser Leu Glu Gly Glu Lys Ala Tyr Arg Glu Thr Thr
   115 120 125
   Asp Leu Gly Ile Glu Pro Leu Arg Ile Gly Ile Lys Lys Leu Asp Glu
   130 135 140
   Asn Ala Ile Asp Asn Tyr Lys Pro Thr Glu Ile Ala Ser Ser Leu Leu
   145 150 155 160
   Val Val Ile Gln Met Val Ser Glu Ala Ala Arg Phe Thr Phe Ile Glu
   165 170 175
   Asn Gln Ile Arg Asn Asn Phe Gln Gln Arg Ile Arg Pro Ala Asn Asn
   180 185 190
   Thr Ile Ser Leu Glu Asn Lys Trp Gly Lys Leu Ser Phe Gln Ile Arg
   195 200 205
   Thr Ser Gly Ala Asn Gly Met Phe Ser Glu Ala Val Glu Leu Glu Arg
   210 215 220
   Ala Asn Gly Lys Lys Tyr Tyr Val Thr Ala Val Asp Gln Val Lys Pro
   225 230 235 240
   Lys Ile Ala Leu Leu Lys Phe Val Asp Lys Asp Pro Glu Gly Thr Cys
   245 250 255
   Tyr Asp Tyr Lys Asp His Asp Gly Asp Tyr Lys Asp His Asp Ile Asp
   260 265 270
   Tyr Lys Asp Asp Asp Asp Lys Ser Arg
   275 280
<210> 10
   <211> 1668
   <212> DNA
   <213> Artificial Sequence
<220>
   <223> Synthetic construct
<400> 10
   catatgtcaa tagttatgac acaagctgca ccatctattc ctgttactcc tggagaatca        60
   gtatcaatct catgtcgttc tagtaaaagt cttctgaata gtaatggtaa cacttactta       120
   tattggttcc tgcaacgtcc aggccaatct cctcaacttc tgatttatcg tatgtcaaac       180
   cttgcttcag gtgttccaga ccgtttcagt ggtagtggtt caggaactgc tttcacactg       240
   agaatcagta gagtagaagc tgaagatgta ggtgtttatt actgtatgca acatttagaa       300
   tatcctctta ctttcggttg tggtacaaaa ctggaaatca aacgaggagg aggaggatct       360
   ggaggaggag gatctggagg aggcggatca ggaggaggtg gttcacaagt tcaacttcaa       420
   caatctggac ctgaactgat taaacctggt gcttcagtaa aaatgtcatg taaagcttct       480
   ggatacacat tcactagcta tgttatgcac tgggtaaaac aaaaacctgg tcaatgtctt       540
   gaatggattg gatatattaa tccttacaat gatggtacta aatacaatga aaaattcaaa       600
   ggtaaagcta cactgacttc agacaaatca tcaagcacag cttacatgga acttagcagc       660
   ctgacatctg aagactctgc agtttattac tgtgcaagag gtacttatta ctacggtagt       720
   cgtgtatttg actactgggg ccaaggtaca actcttacag ttacagtttc atctgcttct       780
   ggtgctggta cctcttcagg tggtggtggt tcaggtggtg gtggttctgg tttagataca       840
   gtttcatttt caacaaaagg tgctacatat attacatacg ttaacttttt aaatgaatta       900
   cgtgttaaat taaaaccaga aggtaattca catggtattc cattattacg taaaaaatgt       960
   gatgatccag gtaaatgttt tgttttagtt gctttatcaa atgataatgg tcaattagct      1020
   gaaattgcta ttgatgttac atcagtttat gttgttggtt atcaagttcg taatcgttca      1080
   tattttttta aagatgctcc agatgctgct tatgaaggtt tatttaaaaa tacaattaaa      1140
   acacgtttac attttggtgg ttcatatcca tcattagaag gtgaaaaagc ttatcgtgaa      1200
   acaacagatc ttggtatcga accacttcgc atcggcatca aaaaacttga cgaaaacgcg      1260
   atcgacaact acaaaccaac agaaatcgcg agctctcttc ttgttgtaat ccaaatggta      1320
   agcgaagcgg cacgtttcac attcatcgaa aaccaaattc gtaacaactt ccaacaacgt      1380
   atccgtccag cgaacaacac aatctctctt gaaaacaaat ggggcaaact tagcttccaa      1440
   atccgtacaa gcggtgcgaa cggtatgttc agcgaagcgg tagaacttga acgcgcgaac      1500
   ggcaaaaaat actacgtaac tgcggtagat caagtaaaac caaaaatcgc acttcttaaa      1560
   ttcgtagaca aagacccaga aggtacctgt tacgattata aagatcacga tggtgattac      1620
   aaagatcacg atattgatta taaagatgat gatgataaat aatctaga                  1668
<210> 11
   <211> 554
   <212> PRT
   <213> Artificial Sequence
<220>
   <223> Synthetic construct
<400> 11
   Met Ser Ile Val Met Thr Gln Ala Ala Pro Ser Ile Pro Val Thr Pro
   1               5                   10                  15
   Gly Glu Ser Val Ser Ile Ser Cys Arg Ser Ser Lys Ser Leu Leu Asn
               20                  25                  30
   Ser Asn Gly Asn Thr Tyr Leu Tyr Trp Phe Leu Gln Arg Pro Gly Gln
   35 40 45
   Ser Pro Gln Leu Leu Ile Tyr Arg Met Ser Asn Leu Ala Ser Gly Val
   50 55 60
   Pro Asp Arg Phe Ser Gly Ser Gly Ser Gly Thr Ala Phe Thr Leu Arg
   65 70 75 80
   Ile Ser Arg Val Glu Ala Glu Asp Val Gly Val Tyr Tyr Cys Met Gln
   85 90 95
   His Leu Glu Tyr Pro Leu Thr Phe Gly Cys Gly Thr Lys Leu Glu Ile
   100 105 110
   Lys Arg Gly Gly Gly Gly Ser Gly Gly Gly Gly Ser Gly Gly Gly Gly
   115 120 125
   Ser Gly Gly Gly Gly Ser Gln Val Gln Leu Gln Gln Ser Gly Pro Glu
   130 135 140
   Leu Ile Lys Pro Gly Ala Ser Val Lys Met Ser Cys Lys Ala Ser Gly
   145 150 155 160
   Tyr Thr Phe Thr Ser Tyr Val Met His Trp Val Lys Gln Lys Pro Gly
   165 170 175
   Gln Cys Leu Glu Trp Ile Gly Tyr Ile Asn Pro Tyr Asn Asp Gly Thr
   180 185 190
   Lys Tyr Asn Glu Lys Phe Lys Gly Lys Ala Thr Leu Thr Ser Asp Lys
   195 200 205
   Ser Ser Ser Thr Ala Tyr Met Glu Leu Ser Ser Leu Thr Ser Glu Asp
   210 215 220
   Ser Ala Val Tyr Tyr Cys Ala Arg Gly Thr Tyr Tyr Tyr Gly Ser Arg
   225 230 235 240
   Val Phe Asp Tyr Trp Gly Gln Gly Thr Thr Leu Thr Val Thr Val Ser
   245 250 255
   Ser Ala Ser Gly Ala Gly Thr Ser Ser Gly Gly Gly Gly Ser Gly Gly
   260 265 270
   Gly Gly Ser Gly Leu Asp Thr Val Ser Phe Ser Thr Lys Gly Ala Thr
   275 280 285
   Tyr Ile Thr Tyr Val Asn Phe Leu Asn Glu Leu Arg Val Lys Leu Lys
   290 295 300
   Pro Glu Gly Asn Ser His Gly Ile Pro Leu Leu Arg Lys Lys Cys Asp
   305                 310                 315                 320
   Asp Pro Gly Lys Cys Phe Val Leu Val Ala Leu Ser Asn Asp Asn Gly
                   325                 330                 335
   Gln Leu Ala Glu Ile Ala Ile Asp Val Thr Ser Val Tyr Val Val Gly
               340                 345                 350
   Tyr Gln Val Arg Asn Arg Ser Tyr Phe Phe Lys Asp Ala Pro Asp Ala
           355                 360                 365
   Ala Tyr Glu Gly Leu Phe Lys Asn Thr Ile Lys Thr Arg Leu His Phe
       370                 375                 380
   Gly Gly Ser Tyr Pro Ser Leu Glu Gly Glu Lys Ala Tyr Arg Glu Thr
   385                 390                 395                 400
   Thr Asp Leu Gly Ile Glu Pro Leu Arg Ile Gly Ile Lys Lys Leu Asp
                   405                 410                 415
   Glu Asn Ala Ile Asp Asn Tyr Lys Pro Thr Glu Ile Ala Ser Ser Leu
               420                 425                 430
   Leu Val Val Ile Gln Met Val Ser Glu Ala Ala Arg Phe Thr Phe Ile
           435                 440                 445
   Glu Asn Gln Ile Arg Asn Asn Phe Gln Gln Arg Ile Arg Pro Ala Asn
       450                 455                 460
   Asn Thr Ile Ser Leu Glu Asn Lys Trp Gly Lys Leu Ser Phe Gln Ile
   465                 470                 475                 480
   Arg Thr Ser Gly Ala Asn Gly Met Phe Ser Glu Ala Val Glu Leu Glu
                   485                 490                 495
   Arg Ala Asn Gly Lys Lys Tyr Tyr Val Thr Ala Val Asp Gln Val Lys
               500                 505                 510
   Pro Lys Ile Ala Leu Leu Lys Phe Val Asp Lys Asp Pro Glu Gly Thr
           515                 520                 525
   Cys Tyr Asp Tyr Lys Asp His Asp Gly Asp Tyr Lys Asp His Asp Ile
       530                 535                 540
   Asp Tyr Lys Asp Asp Asp Asp Lys Ser Arg
   545                 550
<210> 12
   <211> 2400
   <212> DNA
   <213> Artificial Sequence
<220>
   <223> Synthetic construct
<400> 12
   catatgatag ttatgacaca agctgcacca tctattcctg ttactcctgg agaatcagta      60
   tcaatctcat gtcgttctag taaaagtctt ctgaatagta atggtaacac ttacttatat     120
   tggttcctgc aacgtccagg ccaatctcct caacttctga tttatcgtat gtcaaacctt     180
   gcttcaggtg ttccagaccg tttcagtggt agtggttcag gaactgcttt cacactgaga     240
   atcagtagag tagaagctga agatgtaggt gtttattact gtatgcaaca tttagaatat     300
   cctcttactt tcggttgtgg tacaaaactg gaaatcaaac gaggaggagg aggatctgga     360
   ggaggaggat ctggaggagg cggatcagga ggaggtggtt cacaagttca acttcaacaa     420
   tctggacctg aactgattaa acctggtgct tcagtaaaaa tgtcatgtaa agcttctgga     480
   tacacattca ctagctatgt tatgcactgg gtaaaacaaa aacctggtca atgtcttgaa     540
   tggattggat atattaatcc ttacaatgat ggtactaaat acaatgaaaa attcaaaggt     600
   aaagctacac tgacttcaga caaatcatca agcacagctt acatggaact tagcagcctg     660
   acatctgaag actctgcagt ttattactgt gcaagaggta cttattacta cggtagtcgt     720
   gtatttgact actggggcca aggtacaact cttacagtta cagtttcatc tgcttctggt     780
   gctagatctc caaaatcttg tgacaaaact cacacatgtc caccttgtcc agcacctgaa     840
   ctacttggtg gtccttcagt tttcctattc ccaccaaaac caaaagacac actaatgatc     900
   tcacgtacac ctgaagttac atgtgtagta gtagacgtaa gtcacgaaga ccctgaagtt     960
   aaattcaact ggtacgtaga cggtgtagaa gtacataatg caaaaactaa acctcgtgaa    1020
   gaacaataca acagtactta ccgtgtagtt agtgttctaa cagttcttca ccaagactgg    1080
   cttaatggta aagaatacaa atgtaaagtt tcaaacaaag cactaccagc accaatcgaa    1140
   aaaacaatct cacaattggg taccagttct ggtggcggtg gcagtagtgg tggtggcggt    1200
   agtagtggtg gcggtggcat ggcagaaggt ggtagcctag cagctctaac tgctcaccaa    1260
   gcttgtcacc taccgctaga aactttcact cgtcatcgcc aaccgcgcgg ttgggaacaa    1320
   ctagaacaat gtggttatcc ggtacaacgt ctagttgcac tttacctagc tgctcgtcta    1380
   tcttggaacc aagttgacca agtaatccgc aacgcactag caagccctgg tagcggtggt    1440
   gacctaggtg aagctatccg cgaacaaccg gaacaagcac gtctagcact aactctagca    1500
   gcagcagaaa gcgaacgctt cgttcgtcaa ggtactggta acgacgaagc aggtgctgca    1560
   aacgcagacg tagtaagcct aacttgtccg gttgcagcag gtgaatgtgc tggtccggct    1620
   gacagcggtg acgcactact agaacgcaac tatcctactg gtgctgaatt ccttggtgac    1680
   ggtggtgacg ttagcttcag cactcgcggt acgcaaaact ggaccgtgga acgcctactt    1740
   caagctcacc gccaactaga agaacgcggt tatgtattcg ttggttacca cggtactttc    1800
   cttgaagctg ctcaaagcat cgttttcggt ggtgtacgcg ctcgcagcca agaccttgac    1860
   gctatctggc gcggtttcta tatcgcaggt gatccggctc tagcatacgg ttacgcacaa    1920
   gaccaagaac ctgacgcacg cggtcgtatc cgcaacggtg cactactacg tgtttatgta    1980
   ccgcgctcta gcctaccggg tttctaccgc actagcctaa ctctagcagc tccggaagct    2040
   gctggtgaag ttgaacgtct aatcggtcat ccgctaccgc tacgcctaga cgcaatcact    2100
   ggtcctgaag aagaaggtgg tcgcctagaa actattcttg gttggccgct agcagaacgc    2160
   actgtagtaa ttccttctgc tatccctact gacccgcgca acgttggtgg tgaccttgac    2220
   ccgtcaagca tccctgacaa ggaacaagct atcagcgcac taccggacta cgcaagccaa    2280
   cctggtaaac cgccgcgcga agacctaaag ggtaccggtg aaaacttata ctttcaaggt    2340
   tcaggtggtg gtggatctga ttataaagat gatgatgaca aaggaaccgg ttaatctaga    2400
<210> 13
   <211> 799
   <212> PRT
   <213> Artificial Sequence
<220>
   <223> Synthetic construct
<400> 13
   His Met Ile Val Met Thr Gln Ala Ala Pro Ser Ile Pro Val Thr Pro
   1               5                   10                  15
   Gly Glu Ser Val Ser Ile Ser Cys Arg Ser Ser Lys Ser Leu Leu Asn
               20                  25                  30
   Ser Asn Gly Asn Thr Tyr Leu Tyr Trp Phe Leu Gln Arg Pro Gly Gln
           35                  40                  45
   Ser Pro Gln Leu Leu Ile Tyr Arg Met Ser Asn Leu Ala Ser Gly Val
       50                  55                  60
   Pro Asp Arg Phe Ser Gly Ser Gly Ser Gly Thr Ala Phe Thr Leu Arg
   65                  70                  75                  80
   Ile Ser Arg Val Glu Ala Glu Asp Val Gly Val Tyr Tyr Cys Met Gln
                   85                  90                  95
   His Leu Glu Tyr Pro Leu Thr Phe Gly Cys Gly Thr Lys Leu Glu Ile
               100                 105                 110
   Lys Arg Gly Gly Gly Gly Ser Gly Gly Gly Gly Ser Gly Gly Gly Gly
           115                 120                 125
   Ser Gly Gly Gly Gly Ser Gln Val Gln Leu Gln Gln Ser Gly Pro Glu
       130                 135                 140
   Leu Ile Lys Pro Gly Ala Ser Val Lys Met Ser Cys Lys Ala Ser Gly
   145                 150                 155                 160
   Tyr Thr Phe Thr Ser Tyr Val Met His Trp Val Lys Gln Lys Pro Gly
   165 170 175
   Gln Cys Leu Glu Trp Ile Gly Tyr Ile Asn Pro Tyr Asn Asp Gly Thr
   180 185 190
   Lys Tyr Asn Glu Lys Phe Lys Gly Lys Ala Thr Leu Thr Ser Asp Lys
   195 200 205
   Ser Ser Ser Thr Ala Tyr Met Glu Leu Ser Ser Leu Thr Ser Glu Asp
   210 215 220
   Ser Ala Val Tyr Tyr Cys Ala Arg Gly Thr Tyr Tyr Tyr Gly Ser Arg
   225 230 235 240
   Val Phe Asp Tyr Trp Gly Gln Gly Thr Thr Leu Thr Val Thr Val Ser
   245 250 255
   Ser Ala Ser Gly Ala Arg Ser Pro Lys Ser Cys Asp Lys Thr His Thr
   260 265 270
   Cys Pro Pro Cys Pro Ala Pro Glu Leu Leu Gly Gly Pro Ser Val Phe
   275 280 285
   Leu Phe Pro Pro Lys Pro Lys Asp Thr Leu Met Ile Ser Arg Thr Pro
   290 295 300
   Glu Val Thr Cys Val Val Val Asp Val Ser His Glu Asp Pro Glu Val
   305 310 315 320
   Lys Phe Asn Trp Tyr Val Asp Gly Val Glu Val His Asn Ala Lys Thr
   325 330 335
   Lys Pro Arg Glu Glu Gln Tyr Asn Ser Thr Tyr Arg Val Val Ser Val
   340 345 350
   Leu Thr Val Leu His Gln Asp Trp Leu Asn Gly Lys Glu Tyr Lys Cys
   355 360 365
   Lys Val Ser Asn Lys Ala Leu Pro Ala Pro Ile Glu Lys Thr Ile Ser
   370 375 380
   Gln Leu Gly Thr Ser Ser Gly Gly Gly Gly Ser Ser Gly Gly Gly Gly
   385 390 395 400
   Ser Ser Gly Gly Gly Gly Met Ala Glu Gly Gly Ser Leu Ala Ala Leu
   405 410 415
   Thr Ala His Gln Ala Cys His Leu Pro Leu Glu Thr Phe Thr Arg His
   420 425 430
   Arg Gln Pro Arg Gly Trp Glu Gln Leu Glu Gln Cys Gly Tyr Pro Val
           435                 440                 445
   Gln Arg Leu Val Ala Leu Tyr Leu Ala Ala Arg Leu Ser Trp Asn Gln
       450                 455                 460
   Val Asp Gln Val Ile Arg Asn Ala Leu Ala Ser Pro Gly Ser Gly Gly
   465                 470                 475                 480
   Asp Leu Gly Glu Ala Ile Arg Glu Gln Pro Glu Gln Ala Arg Leu Ala
                   485                 490                 495
   Leu Thr Leu Ala Ala Ala Glu Ser Glu Arg Phe Val Arg Gln Gly Thr
               500                 505                 510
   Gly Asn Asp Glu Ala Gly Ala Ala Asn Ala Asp Val Val Ser Leu Thr
           515                 520                 525
   Cys Pro Val Ala Ala Gly Glu Cys Ala Gly Pro Ala Asp Ser Gly Asp
       530                 535                 540
   Ala Leu Leu Glu Arg Asn Tyr Pro Thr Gly Ala Glu Phe Leu Gly Asp
   545                 550                 555                 560
   Gly Gly Asp Val Ser Phe Ser Thr Arg Gly Thr Gln Asn Trp Thr Val
                   565                 570                 575
   Glu Arg Leu Leu Gln Ala His Arg Gln Leu Glu Glu Arg Gly Tyr Val
               580                 585                 590
   Phe Val Gly Tyr His Gly Thr Phe Leu Glu Ala Ala Gln Ser Ile Val
           595                 600                 605
   Phe Gly Gly Val Arg Ala Arg Ser Gln Asp Leu Asp Ala Ile Trp Arg
       610                 615                 620
   Gly Phe Tyr Ile Ala Gly Asp Pro Ala Leu Ala Tyr Gly Tyr Ala Gln
   625                 630                 635                 640
   Asp Gln Glu Pro Asp Ala Arg Gly Arg Ile Arg Asn Gly Ala Leu Leu
                   645                 650                 655
   Arg Val Tyr Val Pro Arg Ser Ser Leu Pro Gly Phe Tyr Arg Thr Ser
               660                 665                 670
   Leu Thr Leu Ala Ala Pro Glu Ala Ala Gly Glu Val Glu Arg Leu Ile
           675                 680                 685
   Gly His Pro Leu Pro Leu Arg Leu Asp Ala Ile Thr Gly Pro Glu Glu
       690                 695                 700
   Glu Gly Gly Arg Leu Glu Thr Ile Leu Gly Trp Pro Leu Ala Glu Arg
   705 710 715 720
   Thr Val Val Ile Pro Ser Ala Ile Pro Thr Asp Pro Arg Asn Val Gly
   725 730 735
   Gly Asp Leu Asp Pro Ser Ser Ile Pro Asp Lys Glu Gln Ala Ile Ser
   740 745 750
   Ala Leu Pro Asp Tyr Ala Ser Gln Pro Gly Lys Pro Pro Arg Glu Asp
   755 760 765
   Leu Lys Gly Thr Gly Glu Asn Leu Tyr Phe Gln Gly Ser Gly Gly Gly
   770 775 780
   Gly Ser Asp Tyr Lys Asp Asp Asp Asp Lys Gly Thr Gly Ser Arg
   785 790 795
<210> 14
   <211> 6
   <212> PRT
   <213> Artificial Sequence
<220>
   <223> Synthetic construct
<400> 14
   His His His His His His
   1 5